# EUROPEAN PATENT SPECIFICATION

(11) **EP 3 050 546 B1**
(45) Date of publication and mention of the grant of the patent: **13.01.2021**
(21) Application number: 14847110.5
(22) Date of filing: 24.09.2014
(51) Int. Cl.: A61F 13/02, A61B 5/01, A61B 5/103, A61M 25/02

(54) **SHEET-SHAPED COVERING**
BLATTFÖRMIGE ABDECKUNG
ÉLÉMENT DE PROTECTION EN FORME DE FEUILLE

(30) Priority: 26.09.2013 JP 2013199128
(43) Date of publication of application: 03.08.2016
(73) Proprietor: The University of Tokyo, Tokyo 113-8654 (JP); Terumo Kabushiki Kaisha, Tokyo 151-0072 (JP)
(72) Inventor: SANADA, Hiromi, Tokyo 113-8654 (JP); MURAYAMA, Ryoko, Tokyo 113-8654 (JP); KOMIYAMA, Chieko, Tokyo 113-8654 (JP); TANABE, Hidenori, Nakakoma-gun Yamanashi 409-3853 (JP)
(74) Representative: Casalonga
(86) International application number: PCT/JP2014/004874
(87) International publication number: WO 2015/045371

(56) References cited:
- WO-A2-01/68163
- JP-A- 2013 523 279
- JP-U- H0 326 796
- US-A- 4 448 204
- US-A- 4 649 923
- US-A1- 2008 146 913

## Description

### TECHNICAL FIELD

This disclosure relates to a sheet-shaped covering for medical use, as it is generally known from US4448204.

### BACKGROUND

A liquid medicine, such as an anticancer drug or the like, is administered into a blood vessel by a catheter or other tubular member inserted into the blood vessel from outside the body.

The tip of the tubular member inserted into the blood vessel may, however, be dislodged from the blood vessel, for example due to movement of a portion of the tubular member that extends outside of the body. The liquid medicine may therefore leak outside of the blood vessel.

In order to resolve such a problem, for example the film dressing disclosed in JP 2010-207300 A (PTL 1) is used to secure the tubular member to the body surface. By being attached so as to cover the insertion opening for the tubular member into the living organism, the film dressing both secures the catheter to the body surface and also suppresses penetration of bacteria and the like through the insertion opening for the tubular member into the living organism.

Even when using a film dressing, however, the tubular member may move for reasons such as the tubular member not being sufficiently secured to the body surface. The risk of the liquid medicine leaking outside of the blood vessel thus remains.

### CITATION LIST

### Patent Literature

PTL 1: JP 2010-207300 A .

### SUMMARY

### (Technical Problem)

The liquid medicine may also leak outside of the blood vessel for reasons other than the above-described case of the tubular member being insufficiently secured to the body surface. If the liquid medicine leaks outside of the blood vessel, not only may the skin or subcutaneous tissue redden or swell, but necrosis may also occur. It is therefore desirable to detect such a leak promptly.

It would therefore be helpful to provide a sheet-shaped covering that, when a liquid medicine leaks outside of a vessel such as a blood vessel, allows prompt detection of the liquid medicine leak.

### (Solution to Problem)

In order to achieve such a sheet-shaped covering, one aspect of this disclosure is a sheet-shaped covering to be attached to a body surface, the sheet-shaped covering including: a transparent section covering an insertion opening in the body surface for a tubular member inserted into a body through the body surface so that a distal end of the tubular member is placed inside the body; and a temperature sensitive section that is positioned near the transparent section, contacts the body surface, and changes color in accordance with temperature.

In another aspect of this disclosure, when the sheet-shaped covering is attached to the body surface so as to cover a portion of a vessel in the body, the temperature sensitive section is preferably positioned in a direction parallel to an extending direction of the vessel with respect to a portion of the transparent section covering the insertion opening.

In another aspect of this disclosure, when the sheet-shaped covering is attached to the body surface so as to cover a portion of a vessel in the body, the temperature sensitive section is preferably positioned in a direction orthogonal to an extending direction of the vessel with respect to a portion of the transparent section covering the insertion opening.

In another aspect of this disclosure, the temperature sensitive section is preferably positioned to surround at least a portion of the transparent section.

In another aspect of this disclosure, when viewing the sheet-shaped covering from a thickness direction, an outline of the temperature sensitive section preferably has at least a portion in common with a portion or all of an outline of the transparent section.

Another aspect of this disclosure preferably further includes a connector connecting the transparent section and the temperature sensitive section.

In another aspect of this disclosure, the connector is preferably a transparent resin sheet, and the resin sheet preferably forms one layer, in a thickness direction of the sheet-shaped covering, of each of the temperature sensitive section and the transparent section.

In another aspect of this disclosure, the temperature sensitive section preferably includes an adhesive section on a contact surface of the temperature sensitive section that contacts the body surface, and the adhesive section is preferably capable of adhering to the body surface.

In another aspect of this disclosure, the transparent section preferably includes a transparent adhesive section on a surface of the transparent section that faces the body surface when the sheet-shaped covering is attached to the body surface.

In another aspect of this disclosure, the temperature sensitive section preferably includes the resin sheet and a temperature sensitive sheet that changes color in accordance with temperature and is layered onto a side of a display surface of the temperature sensitive section with respect to the resin sheet, the display surface positioned at an opposite side from a contact surface that contacts the body surface.

In another aspect of this disclosure, the temperature sensitive sheet preferably includes, on a surface of the temperature sensitive sheet facing the resin sheet, an adhesive section that adheres to the resin sheet.

In another aspect of this disclosure, the temperature sensitive sheet preferably includes a grip capable of being gripped when peeling the adhesive section off the resin sheet.

In another aspect of this disclosure, the adhesive section of the temperature sensitive sheet is designated as a first adhesive section, a second adhesive section capable of adhering to the body surface is preferably disposed on the resin sheet, and an adhesive force of the first adhesive section with respect to the resin sheet is preferably smaller than an adhesive force of the second adhesive section with respect to the body surface.

### (Advantageous Effect)

With this disclosure, when a liquid medicine leaks outside of a blood vessel, the liquid medicine leak can be detected promptly.

### BRIEF DESCRIPTION OF THE DRAWINGS

In the accompanying drawings:
FIG. 1 is a cross-sectional diagram illustrating a sheet-shaped covering 1 according to illustrative example 1 not falling under the scope of the claims in a state of being attached to a body surface;
FIG. 2 illustrates the sheet-shaped covering 1 in a state of being attachable to a body surface;
FIG. 3 illustrates layered structures in the thickness direction of the sheet-shaped covering 1;
FIG. 4 illustrates an example of the layered structure of a transparent section in a sheet-shaped covering according to this disclosure;
FIG. 5 is a cross-sectional diagram illustrating the sheet-shaped covering 1 in a state of being attached to the body surface, with liquid medicine or the like leaking from the distal end of the tubular member to the outside of the outer wall of a vessel;
FIGS. 6(a) and 6(b) illustrate an example of the layered structure of a temperature sensitive section in a sheet-shaped covering according to this disclosure;
FIG. 7 illustrates another example of the layered structure of a temperature sensitive section in a sheet-shaped covering according to this disclosure;
FIG. 8 illustrates an example of the shape of the transparent section, the temperature sensitive section, and an extending section in a sheet-shaped covering according to this disclosure;
FIG. 9 is a cross-sectional diagram illustrating a sheet-shaped covering 11 according to illustrative example 2 not falling under the scope of the claims in a state of being attached to a body surface;
FIG. 10 illustrates a sheet-shaped covering 21 according to illustrative example 3 not falling under the scope of the claims.
FIG. 11 is a cross-sectional diagram illustrating the sheet-shaped covering 21 according to illustrative example 3 not falling under the scope of the claims in a state of being attached to a body surface;
FIG. 12 illustrates an example of the shape of the transparent section, the temperature sensitive section, and an extending section in a sheet-shaped covering according to this disclosure;
FIG. 13 is a cross-sectional diagram illustrating a sheet-shaped covering 31 according to illustrative example 4 not falling under the scope of the claims in a state of being attached to a body surface;
FIG. 14 illustrates layered structures in the thickness direction of the sheet-shaped covering 31 in a state before use;
FIG. 15 illustrates a sheet-shaped covering 41 according to illustrative example 5 not falling under the scope of the claims
FIGS. 16(a) and 16(b) illustrate layered structures in the thickness direction of the sheet-shaped covering 41 in a state of being attachable to a body surface;
FIG. 17 is a cross-sectional diagram illustrating a sheet-shaped covering 51 according to Embodiment 1, in a state of being attached to a body surface; and
FIG. 18 illustrates layered structures in the thickness direction of the sheet-shaped covering 51.

### DETAILED DESCRIPTION

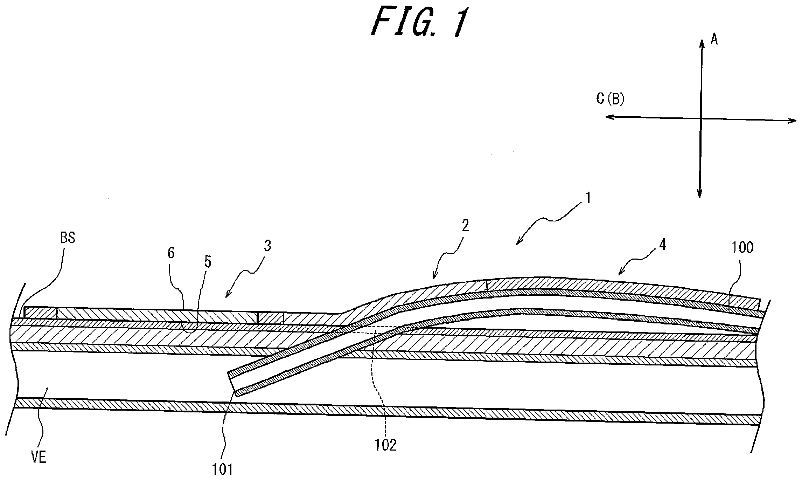

With reference to FIGS. 1 to 18, the following describes illustrative examples not falling under the scope of the claims and embodiments of a sheet-shaped covering for medical use according to this disclosure. Components that are the same between figures are labeled with the same reference signs.

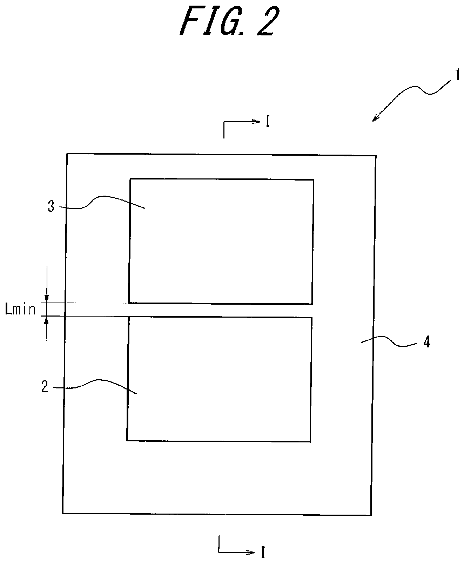

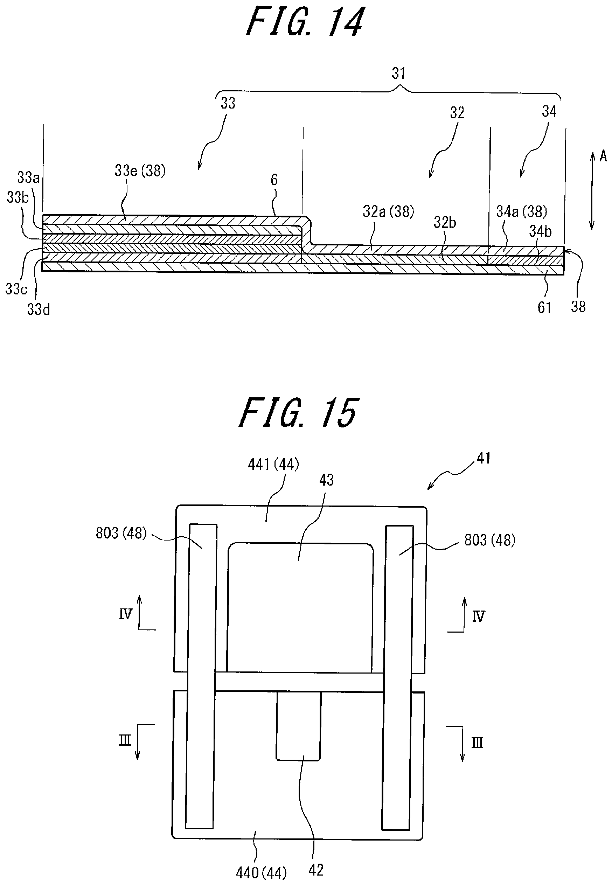

illustrative example 1 not falling under the scope of the claims FIGS. 1 and 2 illustrate a sheet-shaped covering 1, which is illustrative example 1 not falling under the scope of the claims of a sheet-shaped covering for medical use according to this disclosure. Specifically, FIG. 1 is a cross-sectional diagram illustrating the sheet-shaped covering 1 being attached to a body surface so as to cover a portion of a vessel in the body of a living organism. FIG. 2 illustrates the sheet-shaped covering 1 in a state of being attachable to a body surface, as viewed from the thickness direction A of the sheet-shaped covering 1. As used herein, the state of being attachable to a body surface refers to the same state as when the sheet-shaped covering 1 is attached to a body surface and used. In this state, the below-described release layer 61 (see FIG. 14) or the like has been removed. In FIG. 1, "BS" represents the body surface, and "VE" represents the vessel.

As illustrated in FIG. 1, for example when administering a liquid medicine, such as an anticancer drug, or nutrients into a vessel of the body, a tubular member 100 is inserted into the body from the body surface of a living organism through an insertion opening 102 in the body surface. In a state such that the distal end 101 of the tubular member 100 is placed inside the body, the sheet-shaped covering 1 is attached to the body surface so as to cover the insertion opening 102. By attaching the sheet-shaped covering 1 so as to cover the insertion opening 102, penetration of bacteria and the like into the body from the outside through the insertion opening 102 can be suppressed.

As used here, a "vessel" refers to a vessel in the body of a living organism through which bodily fluid passes. Examples include blood vessels and lymph vessels. To administer the above-described anticancer drug or nutrients, the vessel that is normally used is a venous blood vessel in the arm. As used here, a "tubular member" refers to a tube-shaped member with a hollow portion. Examples include an indwelling needle provided with an inner tube and an outer tube; a butterfly needle; and tubes, for administering liquid medicine or the like, that include these needles. Furthermore, the insertion opening for the tubular member in the body surface refers to an opening formed on the body surface by the tubular member puncturing the body surface.

As illustrated in FIG. 2, the sheet-shaped covering 1 includes a transparent section 2, a temperature sensitive section 3 positioned near the transparent section 2 in the extending direction B of the sheet-shaped covering 1 (in the plane of the sheet when viewing the sheet-shaped covering 1 from the thickness direction A), and an extending section 4 other than the transparent section 2 and the temperature sensitive section 3.

As illustrated in FIG. 1, the sheet-shaped covering 1 is attached to the body surface so that the transparent section 2 at least covers the insertion opening 102 for the tubular member 100 in the body surface. In the state in which the sheet-shaped covering 1 is attached to the body surface so as to cover a portion of a vessel in the body, the temperature sensitive section 3 is positioned with respect to at least a portion of the transparent section 2 in a direction C substantially parallel to the extending direction of the vessel and is in contact with the body surface.

The temperature sensitive section 3 is a section that changes color in response to temperature. The temperature sensitive section 3 changes color in response to a change in temperature of the body surface. Therefore, when a change in temperature of the body surface occurs due to a liquid medicine such as an anticancer drug, nutrients, or the like being administered into the body, the temperature sensitive section 3 can render the change in temperature visible as a change in color.

The following describes the configuration of each portion of the sheet-shaped covering 1 and other characteristic features in detail.

### [Transparent Section 2]

The transparent section 2 is a region that is transparent in the thickness direction A when the sheet-shaped covering 1 in a state of being attachable to the body surface is viewed from the thickness direction A. In greater detail, when the sheet-shaped covering 1 in this state is viewed from the direction A, the transparent section 2 is a region formed by a member having optical transparency in the thickness direction A. Since the transparent section 2 is attached to the body surface so as to cover the insertion opening 102 for the tubular member 100, the transparent section 2 can cover and protect the insertion opening 102 while also allowing a user, such as a health professional, to visually confirm the insertion opening 102 from the outside.

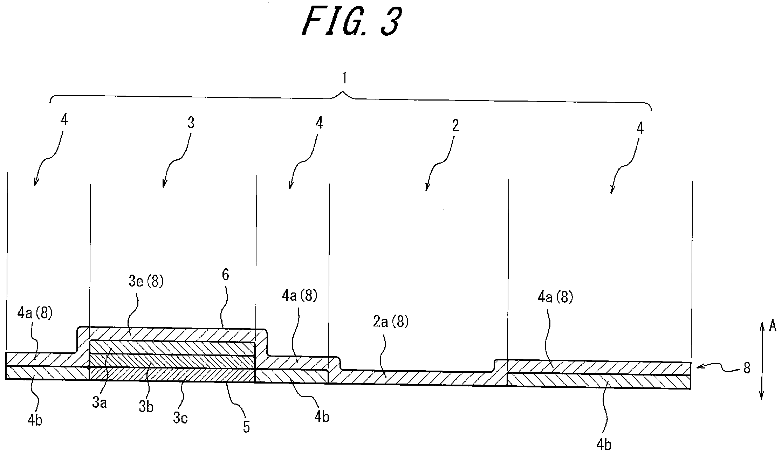

FIG. 3 is a cross-sectional diagram along the I-I line in FIG. 2, illustrating layered structures in the thickness direction A of the sheet-shaped covering 1 in detail. While the layered structures of the transparent section 2, temperature sensitive section 3, and extending section 4 in the thickness direction A are omitted in FIG. 1 for the sake of convenience, these components actually have the layered structures illustrated in FIG. 3. Furthermore, FIG. 3 illustrates the relationship between the layers at each position but does not represent the actual thickness of each layer at each position. The actual thickness of each layer may be set appropriately in accordance with purpose or use.

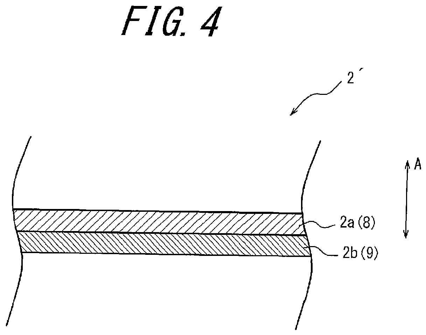

As illustrated in FIG. 3, the transparent section 2 in this illustrative example not falling under the scope of the claims is configured with a sheet-shaped, transparent resin layer 2a. In this illustrative example not falling under the scope of the claims polyurethane is used as the material for the resin layer 2a, but the resin layer 2a is not limited to this material. For example, an acrylic polymer, polyethylene, an ethylene-vinyl acetate copolymer, polyether polyester, a polyamide derivative, or the like may be used. An acrylic polymer, polyurethane, polyether polyester, and a polyamide derivative are particularly suitable as the material for the resin layer 2a as these materials have both excellent water vapor permeability and high transparency. The transparent section 2 in this illustrative example not falling under the scope of the claims embodiment is a single layer structure containing the resin layer 2a. A multilayered transparent section 2' may, however, be used. For example as illustrated in FIG. 4, the transparent section 2' includes the resin layer 2a and an adhesive layer 2b, which is a transparent adhesive section 9 layered onto the surface of the resin layer 2a facing the body surface.

By adopting a configuration that includes the adhesive layer 2b, the adhesive layer 2b contacts the body surface around the insertion opening 102 and adheres the transparent section 2 to the body surface, thereby suppressing movement of a portion of the tubular member 100 extending outside of the body near the insertion opening 102. Furthermore, the penetration of bacteria and the like into the body from the outside through the insertion opening 102 can be further suppressed as compared to a configuration that does not include the adhesive layer 2b. The adhesive layer 2b may be provided on a portion of a surface of the resin layer 2a. The transparent adhesive used here may be a rubber adhesive, an acrylic adhesive, a silicone adhesive, or the like, but an acrylic adhesive with high transparency is preferably used.

The resin layer 2a of the transparent section 2 in this illustrative example not falling under the scope of the claims is a portion of a connector 8. The connector 8 is described below in detail.

While the transparent section 2 in this illustrative example not falling under the scope of the claims is colorless and transparent, the transparent section 2 may be colored. In other words, a colored transparent section 2 that absorbs only a portion of visible light while transmitting other light may be used.

Furthermore, although the transparent section 2 in this illustrative example not falling under the scope of the claims is quadrilateral when a surface of the sheet-shaped covering 1 is viewed from the thickness direction A, as illustrated in FIG. 2, the transparent section 2 may have any other planar shape, including a triangle or other polygon, a circle, or an ellipse.

The thickness of the transparent section 2 in this illustrative example not falling under the scope of the claims is set to a range of 5 µm to 150 µm considering the unevenness of the body surface of the living organism near the insertion opening 102 and the ability to follow the shape of the portion of the tubular member 100 extending outside of the body. Any thickness, however, may be adopted in accordance with the material characteristics, length, and the like of the tubular member 100 and with actual usage conditions.

Unlike the below-described temperature sensitive section 3, the transparent section 2 in this illustrative example not falling under the scope of the claims does not have a temperature sensitive property of changing color in accordance with temperature. Accordingly, in a predetermined temperature range over which the temperature sensitive section 3 changes color in accordance with temperature, the transparent section 2 in this illustrative example not falling under the scope of the claims remains colorless and transparent. The temperature sensitive property of the temperature sensitive section 3 is described below.

### [Temperature Sensitive Section 3]

The temperature sensitive section 3 is a region, when the sheet-shaped covering 1 in a state of being attachable to the body surface is viewed from the thickness direction A, that changes color in accordance with temperature within a predetermined temperature range. Specifically, the temperature sensitive section 3 includes a contact surface 5 in contact with the body surface and a display surface 6 on the opposite side from the contact surface 5. In a state such that the contact surface 5 is in contact with the body surface, the display surface 6 displays a color in accordance with the temperature of the body surface detected at the contact surface 5. Accordingly, a user such as a health professional can observe a temperature change in the body surface from the outside via the display surface 6 of the temperature sensitive section 3.

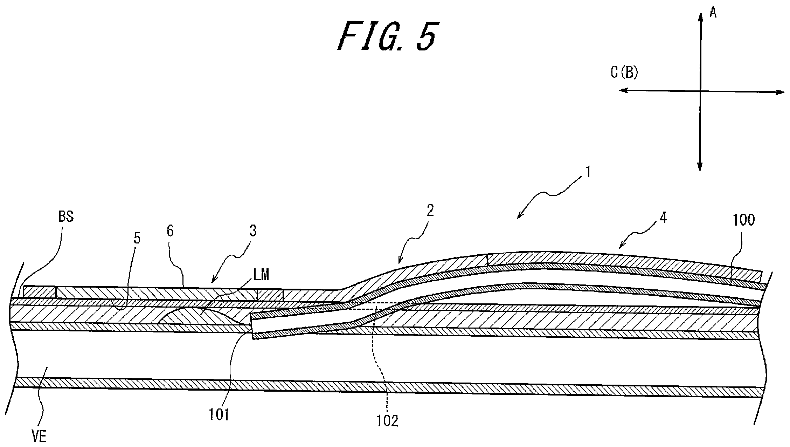

FIG. 5 illustrates the same cross-section as FIG. 1, but the position of the distal end 101 of the tubular member 100 differs from FIG. 1. For example, as illustrated in FIG. 5, the distal end 101 of the tubular member 100 is not completely positioned inside the vessel, and when the liquid medicine or the like, such as an anticancer drug, that should be administered into the vessel from the tubular member 100 leaks from the vessel, the temperature of the body surface near the distal end of the tubular member 100 changes due to the leaking liquid medicine or the like (indicated by "LM" in FIG. 5). Therefore, the change in temperature is detected by the contact surface 5 of the temperature sensitive section 3, and the temperature change is displayed as a change in color by the display surface 6. Accordingly, for example a user such as a health professional can promptly confirm via the temperature sensitive section 3 whether liquid medicine or the like has leaked from the vessel. As used herein, the concept of a "change in color" refers not only to changing from one color to another color, but also to becoming colored, i.e. changing from colorless to colored, and to losing color, i.e. changing from colored to colorless.

Depending on the rate of administration of the liquid medicine or the like, for example with a relatively high rate of administration, a change in temperature in the body surface occurs along the extending direction of the vessel when the liquid medicine or the like is being properly administered into the vessel. Therefore, in addition to confirming whether the liquid medicine has leaked outside the vessel, it can also be confirmed via the temperature sensitive section 3 whether the liquid medicine is being properly administered into the vessel.

Next, the layered structure of the temperature sensitive section 3 in the thickness direction A is described. As illustrated in FIG. 3, the temperature sensitive section 3 in this illustrative example not falling under the scope of the claims is configured by layering a plurality of layers in the thickness direction A.

As illustrated in FIG. 3, the temperature sensitive section 3 in this illustrative example not falling under the scope of the claims includes the following in the thickness direction A: a substrate layer 3a; a temperature sensitive layer 3b that includes cholesteric liquid crystals as a temperature sensitive material and is layered onto one surface of the substrate layer 3a; a black layer 3c, serving as a dark color layer, layered onto the temperature sensitive layer 3b on the opposite side from the substrate layer 3a; and a surface layer 3e layered onto the substrate layer 3a on the opposite side from the temperature sensitive layer 3b and the black layer 3c. In this illustrative example not falling under the scope of the claims the black layer 3c, the temperature sensitive layer 3b, the substrate layer 3a, and the surface layer 3e are layered in this order from the contact surface 5 towards the display surface 6. As used herein, a "dark color" refers to a color with a low level of brightness, such as black, dark green, navy blue, brown, or the like.

The substrate layer 3a in this illustrative example not falling under the scope of the claims is configured with thin, transparent, sheet-shaped polyester. The material of the substrate layer 3a is not limited to the polyester used in this illustrative example not falling under the scope of the claims however, and may, for example, be polyamide, polyamide-imide, polyethylene, polypropylene, polycarbonate, polyvinyl chloride, polyurethane, a fluoropolymer, or the like.

The thickness of the substrate layer 3a is set in a range of 5 µm to 200 µm to allow the temperature sensitive section 3 to be flexible enough to follow the unevenness and the shape of the body surface.

The temperature sensitive layer 3b includes cholesterie liquid crystals as a temperature sensitive material. Cholesteric liquid crystals have a spiral shape. Since the spiral pitch changes in accordance with temperature, the wavelength of strongly reflected light also changes. Accordingly, in the wavelength range of visible light, the wavelength of visible light that is strongly reflected changes in response to temperature. The color that is visually perceived therefore also changes in a reversible manner in response to temperature.

The temperature sensitive layer 3b is configured with ink formed by microencapsulating and dispersing the above-described cholesteric liquid crystals in a suitable binder. In other words, the temperature sensitive layer 3b is formed by applying this ink onto one surface of the substrate layer 3a. In this case, the surface of the substrate layer 3a onto which the ink is applied is the surface of the substrate layer 3a on the side of the contact surface 5.

The thickness of the temperature sensitive layer 3b in this illustrative example not falling under the scope of the claims is set in a range of 5 µm to 100 µm in consideration of sufficient flexibility for following the unevenness and the shape of the body surface.

The black layer 3c emphasizes an interference color due to light that is selectively reflected at the temperature sensitive layer 3b to make the color, and the change thereto, observed at the display surface 6 more vivid. In this illustrative example not falling under the scope of the claims the black layer 3c is formed by applying or printing black paint or ink onto the temperature sensitive layer 3b to form a layer. The black layer 3c may, however, be formed for example by layering a black resin sheet onto the temperature sensitive layer 3b. The thickness of the black layer 3c is preferably set in a range of 3 µm to 100 µm in consideration of sufficient flexibility for following the unevenness and the shape of the body surface. The temperature sensitive section 3 in this illustrative example not falling under the scope of the claims includes the opaque black layer 3c and therefore always has an opaque structure regardless of whether the temperature is within the predetermined temperature range in which the change in color is obervable.

The surface layer 3e is a transparent resin layer with a thickness of approximately 5 µm to 50 µm. Polyester, polyamide, polyamide-imide, polyethylene, polypropylene, polycarbonate, polyurethane, polyvinyl chloride, a fluoropplymer, or the like may be used as the material of the surface layer 3e. In this illustrative example not falling under the scope of the claims polyurethane is used as the material of the surface layer 3e, and one surface of the surface layer 3e constitutes the display surface 6 of the temperature sensitive section 3.

Like the above-described resin layer 2a of the transparent section 2, the surface layer 3e in this illustrative example not falling under the scope of the claims is a portion of a connector 8. The connector 8 is described below in detail.

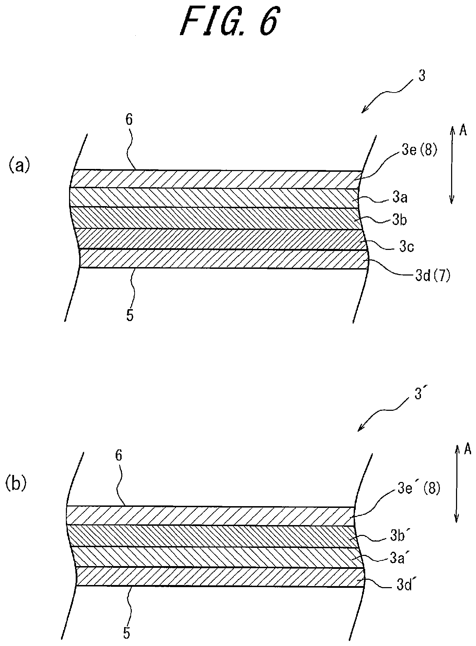

The temperature sensitive section 3 in this illustrative example not falling under the scope of the claims has the above-described four-layer structure. One surface of the black layer 3c constitutes the contact surface 5, and one surface of the surface layer 3e constitutes the display surface 6. The temperature sensitive section 3 is not, however, limited to the above configuration, and for example as illustrated in FIG. 6(a), an adhesive layer 3d as an adhesive section 7 may be further layered onto the black layer 3c on the opposite side from the temperature sensitive layer 3b and the substrate layer 3a. When adopting such a configuration, the adhesive layer 3d directly contacts the body surface, making it easier to secure the sheet-shaped covering 1 onto the body surface. Furthermore, with this configuration, one surface of the adhesive layer 3d constitutes the contact surface 5 of the temperature sensitive section 3. The adhesive layer 3d is configured with adhesive. For example, a rubber adhesive, an acrylic adhesive, a silicone adhesive, or the like may be used. While the adhesive layer 3d serving as the adhesive section 7 is provided on the entire contact surface 5 that contacts the body surface in FIG. 6(a), the adhesive layer 3d may instead be provided on a portion of the contact surface 5.

Furthermore, the temperature sensitive section 3 in this illustrative example not falling under the scope of the claims is configured so that the black layer 3c, the temperature sensitive layer 3b, the substrate layer 3a, and the surface layer 3e are layered in this order from the contact surface 5 towards the display surface 6, but the temperature sensitive section 3 is not limited to this configuration. For example as illustrated in FIG. 6(b), a temperature sensitive section 3' in which an adhesive layer 3d' serving as the adhesive section 7, a substrate layer 3a', a temperature sensitive layer 3b', and a surface layer 3e' (a portion of the connector 8) serving as a finishing layer are layered in this order from the contact surface 5 towards the display surface 6 may be adopted. When adopting such a configuration, the substrate layer 3a' preferably has a thickness of 5 µm to 100 µm so that the temperature of the body surface can be rapidly transmitted to the temperature sensitive layer 3b'. Furthermore, in order to emphasize the interference with light selectively reflected by the temperature sensitive layer 3b', the substrate layer 3a' is preferably configured with a resin material of a dark color such as black. If the substrate layer 3a' itself is formed from a resin material of a dark color, a separate black layer need not be provided, thereby simplifying the configuration of the temperature sensitive section 3'. Making the structure thinner also improves flexibility and thermal conductivity. The adhesive layer 3d' and the surface layer 3e' are similar to the above-described adhesive layer 3d and surface layer 3e.

Although cholesteric liquid crystals are used as the temperature sensitive material in this illustrative example not falling under the scope of the claims for example as an inorganic material, a metal complex salt such as Ag₂HgI₄, Cu₂HgI₄, or HgI₂ may be used, and as an organic material, condensed aromatic ring compounds such as spiropyrans, substituted ethylene derivatives such as anthrones, and lactone ring compounds such as crystal violet lactone may be used.

Processing methods that may be used to provide the temperature sensitive layer 3b that includes temperature sensitive material on one surface of the substrate layer 3a include not only the above-described method in this illustrative example not falling under the scope of the claims, but also for example a method for applying the temperature sensitive material directly on one surface of the substrate layer 3a, and a method for forming ink from the temperature sensitive material using a suitable binder and printing the ink on the substrate layer 3a.

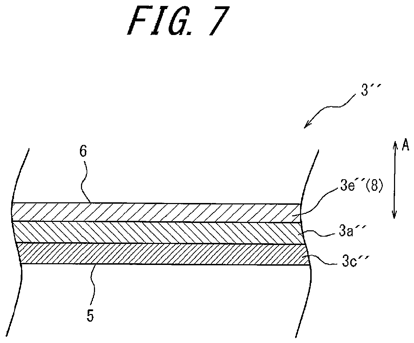

In addition to the configurations, illustrated in this illustrative example not falling under the scope of the claims of the temperature sensitive sections 3 and 3', in which the substrate layers 3a and 3a' and the temperature sensitive layers 3b and 3b' are layered, a temperature sensitive section 3" may also be adopted. As illustrated in FIG. 7, the temperature sensitive section 3" includes a substrate layer 3a" including temperature sensitive material, a surface layer 3e" layered onto one surface of the substrate layer 3a", and a black layer 3c" layered onto the substrate layer 3a" on the opposite side from the surface layer 3e". The black layer 3c", the substrate layer 3a", and the surface layer 3e" are layered in this order from the contact surface 5 towards the display surface 6. The substrate layer 3a" is a layer retaining a temperature sensitive material, such as cholesteric liquid crystals, in the matrix of a polymeric material. The configuration of the surface layer 3e" and the black layer 3c" in the temperature sensitive section 3" is similar to that of the surface layer 3e and the black layer 3c in the temperature sensitive section 3 in this illustrative example not falling under the scope of the claims and the surface layer 3e" is a portion of the below-described connector 8. An adhesive layer may also be layered onto the black layer 3c" of the temperature sensitive section 3".

Next, the predetermined temperature range over which the temperature sensitive section 3 in this illustrative example not falling under the scope of the claims changes color is described. The temperature sensitive section 3 in this illustrative example not falling under the scope of the claims is configured to change color in a predetermined temperature range of 28°C to 38°C. Specifically, when viewing the display surface 6 of the temperature sensitive section 3 from the thickness direction A, the color of the temperature sensitive section 3 that is observed at the display surface 6 is black when the temperature detected by the temperature sensitive layer 3b is less than 28°C. As the temperature detected by the temperature sensitive layer 3b rises from 28°C to 38°C, the color changes in the following order: red, orange, yellow, green, and blue. When the temperature detected by the temperature sensitive layer 3b exceeds 38°C, the color that is displayed remains blue. The color of the temperature sensitive section 3 changes in a reversible manner, so that when the temperature detected by the temperature sensitive layer 3b falls from 38°C, the color again changes in the following order: blue, green, yellow, orange, red, and black.

The above-described predetermined temperature range may be set appropriately by mixing different types of cholesteric liquid crystals. Therefore, considering how the change in temperature of the body surface occurring due to liquid medicine or nutrients leaking outside the vessel can be expressed to a high degree of accuracy as a change in color at the display surface 6, the predetermined temperature range is particularly preferably from 31°C to 35°C. By setting the predetermined temperature range in the above-mentioned range, the skin surface temperature of the forearm (approximately 33°C) falls within this range, so that a change in temperature of the body surface due to leaking of liquid medicine or the like administered at a lower temperature (room temperature) can be displayed to a higher degree of accuracy.

The predetermined temperature range over which the color of the temperature sensitive section 3 changes is not limited to the above-described range and may be set appropriately in accordance with purpose or use. The reason is that the skin surface temperature varies by season, the environment in which the liquid medicine or the like is administered, the administration site, and the patient's pathological condition, such as sensitivity to cold. In this illustrative example not falling under the scope of the claims the temperature sensitive section displays black at a temperature below the predetermined temperature range and displays blue at a higher temperature than the predetermined temperature range, but by changing the type of temperature sensitive material or the method of forming the temperature sensitive section, the temperature sensitive section may be configured to be colorless outside of the predetermined temperature range and colored within the predetermined temperature range. Furthermore, when confirmation of a change in color in a plurality of different temperature ranges is desired, the temperature sensitive section may be formed by mixing a plurality of temperature sensitive materials that have different predetermined temperature ranges over which the color changes.

In this way, the temperature sensitive section 3 may be implemented with a variety of known methods and is not limited to the configuration described in this illustrative example not falling under the scope of the claims.

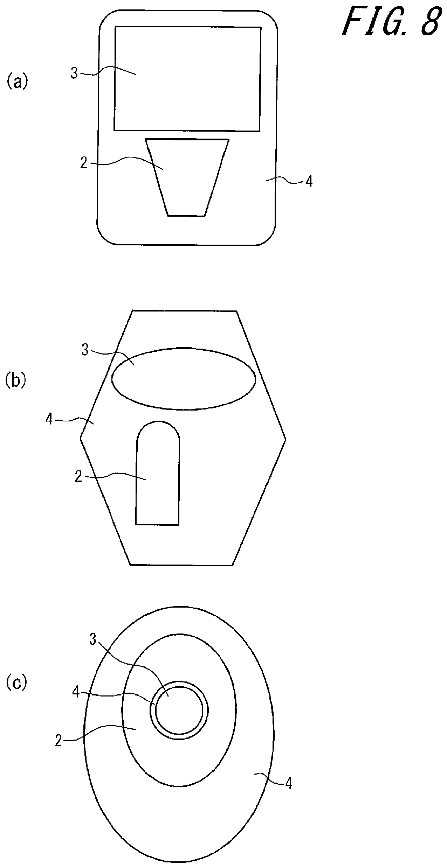

Although the temperature sensitive section 3 in this illustrative example not falling under the scope of the claims is quadrilateral when viewed from the thickness direction A, a variety of shapes are possible depending on the positional relationship with the transparent section 2, as described below (see FIGS. 8(a) to 8(c)).

### [Extending Section 4]

The extending section 4 is the area other than the transparent section 2 and the temperature sensitive section 3 when the sheet-shaped covering 1 is viewed from the thickness direction A. As illustrated in FIG. 3, the extending section 4 in this illustrative example not falling under the scope of the claims includes a transparent resin layer 4a and an adhesive layer 4b layered onto the resin layer 4a. The adhesive layer 4b and the resin layer 4a are layered in this order from the surface that faces the body surface when the sheet-shaped covering I is attached to the body surface.

In the transparent resin layer 4a, the same material as the material used in the above-described transparent section 2 may be used. In this illustrative example not falling under the scope of the claims polyurethane resin is used. The adhesive layer 4b in this illustrative example not falling under the scope of the claims is an opaque cloth tape configured with non-woven fabric. Since the above-described opaque cloth tape is used as the adhesive layer 4b, the extending section 4 in this illustrative example not falling under the scope of the claims does not form part of the transparent section 2.

Like the above-described resin layer 2a of the transparent section 2 and surface layer 3e of the temperature sensitive section 3, the transparent resin layer 4a is a portion of the below-described connector 8. The connector 8 is described below in detail.

### [Connector 8]

Next, the connection between the transparent section 2 and the temperature sensitive section 3 is described with reference to FIG. 3. The sheet-shaped covering 1 includes the connector 8 that connects the transparent section 2 and the temperature sensitive section 3. The connector 8 in this illustrative example not falling under the scope of the claims is a transparent resin sheet. As illustrated in FIG. 3, the connector 8 constitutes one layer of the transparent section 2, the temperature sensitive section 3, and the extending section 4 in the thickness direction A. Specifically, in this illustrative example not falling under the scope of the claims the transparent section 2 is a single layer structure constituted by the transparent resin layer 2a, and this resin layer 2a is a portion of the connector 8. In the temperature sensitive section 3, the surface layer 3e is a portion of the connector 8. Furthermore, in the extending section 4, the resin layer 4a is a portion of the connector 8. In this way, the transparent section 2, temperature sensitive section 3, and extending section 4 in this illustrative example not falling under the scope of the claims are integrated by each including the common connector 8 (2a, 3e, and 4a) as one layer thereof in the thickness direction A.

The transparent, thin resin sheet used as the connector 8 in this illustrative example not falling under the scope of the claims is, for example, configured with polyurethane, and the thickness thereof is set in a range of 5 µm to 150 µm. The material of the connector 8 is not, however, limited to polyurethane. For example, an acrylic polymer, polyethylene, an ethylene-vinyl acetate copolymer, polyether polyester, a polyamide derivative, or the like may be used.

The connector 8 (2a, 3e, and 4a) and the other layers are connected by, for example, being adhered with an adhesive or fused together.

The connector 8 in this illustrative example not falling under the scope of the claims extends across the entire area of one surface of each of the transparent section 2, temperature sensitive section 3, and extending section 4, but the connector 8 may, for example, be layered in only a portion of each section in the extending direction B. Furthermore, in this illustrative example not falling under the scope of the claims the connector 8 constitutes the resin layer 2a of the transparent section 2, the surface layer 3e of the temperature sensitive section 3, and the resin layer 4a of the extending section 4, but the connector 8 is not limited to these layers. For example, instead of the surface layer 3e of the temperature sensitive section 3, the connector 8 may constitute the substrate layer 3 a.

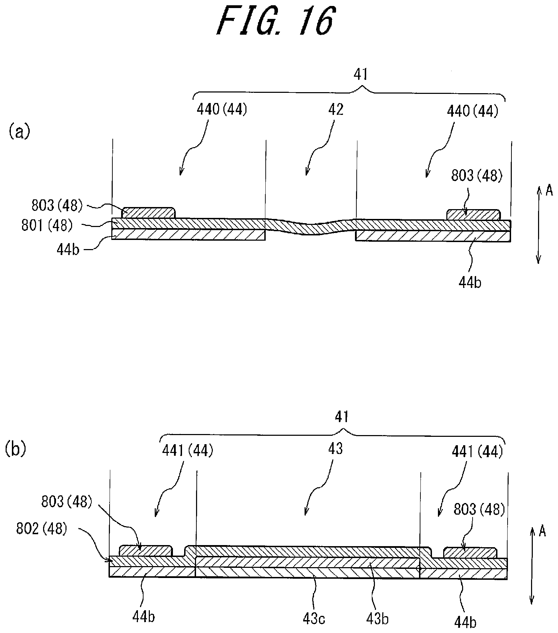

In this illustrative example not falling under the scope of the claims the single-layer connector 8 constitutes all of the resin layer 2a of the transparent section 2, the surface layer 3e of the temperature sensitive section 3, and the resin layer 4a of the extending section 4, but instead, a plurality of connectors may be used to form a composite connector by connecting the plurality of connectors to each other. Details on a composite connector 48 are described below as illustrative example 5 not falling under the scope of the claims (see FIGS. 15, 16(a), and 16(b)).

### [Positional Relationship Between Transparent Section 2 and Temperature Sensitive Section 3]

Thus far, the configuration of each section of the sheet-shaped covering 1 has been described, Next, as one of the features of the sheet-shaped covering 1, the positional relationship between the transparent section 2 and the temperature sensitive section 3 when viewing the sheet-shaped covering 1 from the thickness direction A is described.

The transparent section 2 and the temperature sensitive section 3 are positioned near each other in the extending direction B of the sheet-shaped covering 1. By adopting such an arrangement; the temperature sensitive section 3 can be contacted to the body surface near the distal end 101 of the tubular member 100 that is inserted through the insertion opening 102 covered by the transparent section 2.

In this illustrative example not falling under the scope of the claims as illustrated in FIG. 2, the minimum distance Lmin between the transparent section 2 and the temperature sensitive section 3 is set to 5 mm, taking into consideration the length of the portion of the tubular member 100 that extends into the body through the insertion opening 102 (see FIGS. 1 and 5). Along the tubular member 100, the length of the portion that extends into the body through the insertion opening 102 normally is approximately 20 mm to 30 mm when, for example, administering an anticancer drug as the liquid medicine through a venous blood vessel in the arm as the vessel. Therefore, if the minimum distance Lmin between the transparent section 2 and the temperature sensitive section 3 is set to 20 mm or less, a health professional can observe, via the temperature sensitive section 3, whether the anticancer drug has leaked from the venous blood vessel. Specifically, if the transparent section 2 and the temperature sensitive section 3 are attached to the body surface so as to lie along the extending direction of the venous blood vessel in the arm, with a position in the transparent section 2 near the temperature sensitive section 3 covering the insertion opening 102, then since the distal end 101 of the tubular member 100 reaches a location by the temperature sensitive section 3, leaking of the anticancer drug can be detected with the temperature sensitive section 3.

Along the tubular member 100, the length of the portion that extends into the body through the insertion opening 102 varies by factors such as the type of tubular member and the position on the body surface at which the tubular member is inserted. Therefore, the minimum distance Lmin can be set larger than 20 mm in a range over which a change in temperature of the body surface due to liquid medicine or the like can be detected in the temperature sensitive section 3. Considering a variety of usage conditions, however, the minimum distance Lmin is preferably set to 20 mm or less.

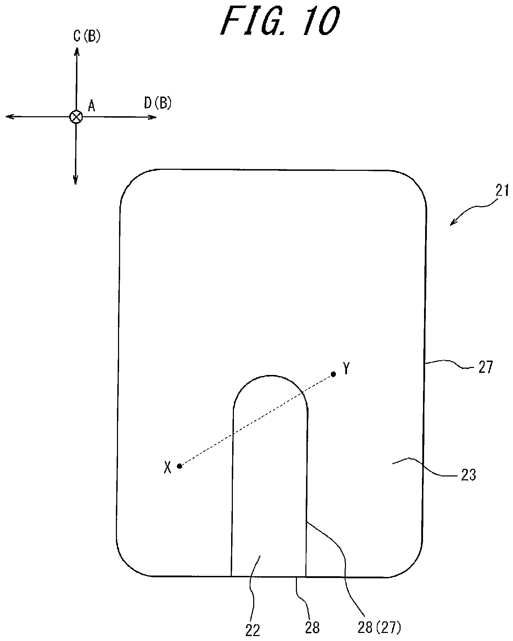

The position in the transparent section 2 that actually covers the insertion opening 102, however, varies depending on the user that handles the sheet-shaped covering 1. Depending on this position, the position of the distal end 101 of the tubular member 100 with respect to the transparent section 2 changes. Hence, the minimum distance Lmin is most preferably set to 0 mm to dispose the transparent section 2 and the temperature sensitive section 3 next to each other. Details on a configuration in which the transparent section 2 and the temperature sensitive section 3 are disposed next to each other are described below (for example, see FIG. 10).

Furthermore, in this illustrative example not falling under the scope of the claims in a state in which the sheet-shaped covering 1 is attached to the body surface so as to cover a portion of a vessel in the body of the living organism, the temperature sensitive section 3 is positioned, with respect to the portion of the transparent section 2 covering the insertion opening, in a direction C that is substantially parallel to the extending direction of the vessel among the extending directions B of the sheet-shaped covering 1, as illustrated in FIG. 1. In other words, when the sheet-shaped covering 1 is viewed from the thickness direction A, at least a portion of the transparent section 2 is aligned with at least a portion of the temperature sensitive section 3 along the extending direction of the vessel.

The transparent section 2 at least covers the insertion opening 102 in the body surface, and along the tubular member 100, the portion that extends into the body through the insertion opening 102 extends along the extending direction of the vessel. Accordingly, with respect to the portion of the transparent section 2 that covers the insertion opening 102, the distal end 101 of the tubular member 100 is positioned in a direction substantially parallel to the extending direction of the vessel. Therefore, with respect to the portion of the transparent section 2 covering the insertion opening 102, if the temperature sensitive section 3 is positioned at least in the direction C parallel to the extending direction of the vessel, then even if the distal end 101 of the tubular member 100 is outside of the vessel, the temperature sensitive section 3 is more likely to be positioned near the distal end 101 of the tubular member 100. Hence, this arrangement is preferred.

In this illustrative example not falling under the scope of the claims, when the sheet-shaped covering 1 is viewed from the thickness direction A, the transparent section 2 and the temperature sensitive section 3 are both quadrilateral, yet the shape of the transparent section 2 and the temperature sensitive section 3 is not limited to being quadrilateral. As long as the temperature sensitive section 3 can be attached to the body surface so as to be positioned at least in the direction C parallel to the extending direction of the vessel with respect to the portion of the transparent section 2 that covers the insertion opening 102, then the shape of the transparent section 2 and the temperature sensitive section 3 as viewed from the thickness direction A may be any shape. For example, as illustrated in FIG. 8(a), the transparent section 2 may be trapezoidal, and the temperature sensitive section 3 may be rectangular, or as illustrated in FIG. 8(b), the transparent section 2 may have a substantially rectangular shape, the outline of which is curved at one end in the parallel direction C, and the temperature sensitive section 3 may be elliptical. As illustrated in FIG. 8(c), the transparent section 2 may be annular, with a circular temperature sensitive section 3 being surrounded by the transparent section 2. The position of the transparent section 2 and the temperature sensitive section 3 within the entire sheet-shaped covering 1 may be set appropriately in accordance with purpose or use, so long as the transparent section 2 and the temperature sensitive section 3 satisfy the above-described positional relationship, i.e. the positional relationship whereby, with respect to the portion of the transparent section 2 covering the insertion opening 102, the temperature sensitive section 3 is positioned at least in the direction C parallel to the extending direction of the vessel. Accordingly, as illustrated in FIG. 8(b), the transparent section 2 and the temperature sensitive section 3 may be arranged so that no common axis of symmetry about which the transparent section 2 and the temperature sensitive section 3 are symmetrical exists.

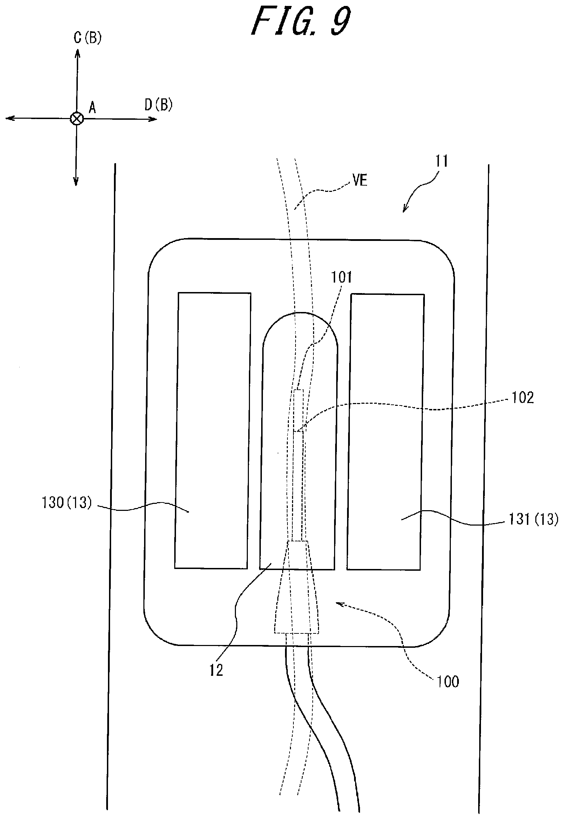

In this illustrative example not falling under the scope of the claims, as illustrated in FIG. 2, the shape of the sheet-shaped covering 1 when viewing the sheet-shaped covering 1 from the thickness direction A (the same shape as the outline of the outer edge of the extending section 4 in this illustrative example not falling under the scope of the claims) is quadrilateral, yet this configuration is not limiting. For example, the shape may be a substantially quadrilateral shape in which each corner is curved as illustrated in FIG. 8(a), a hexagonal shape as illustrated in FIG. 8(b), or an elliptical shape as illustrated in FIG. 8(c),

illustrative example 2 not falling under the scope of the claims Next, a sheet-shaped covering 11 according to illustrative example 2 not falling under the scope of the claims of this disclosure is described. FIG. 9 illustrates the sheet-shaped covering 11 attached to a body surface so as to cover a portion of a vessel in the body, as viewed from the thickness direction A of the sheet-shaped covering 11. Like the above-described sheet-shaped covering 1 according to illustrative example 1 not falling under the scope of the claims, the sheet-shaped covering 11 includes a transparent section 12 and a temperature sensitive section 13 positioned near the transparent section 12 in the extending direction B of the sheet-shaped covering 11. The dashed lines in FIG. 9 represent a vessel VE in the body, specifically a venous blood vessel in the arm.

In this illustrative example not falling under the scope of the claims in a state in which the sheet-shaped covering 11 is attached to the body surface so as to cover a portion of a vessel in the body, the temperature sensitive section 13 is positioned, with respect to a portion of the transparent section 12 that covers the insertion opening 102, at least in a direction D that, among the extending directions B of the sheet-shaped covering 11, is orthogonal to the extending direction of the vessel ("orthogonal direction D"). As compared to the sheet-shaped covering 1 according to illustrative example 1 not falling under the scope of the claims in a state of attachment to the body surface, the positional relationship of the transparent section and the temperature sensitive section with respect to the extending direction of the vessel differs in the sheet-shaped covering 11 according to this illustrative example not falling under the scope of the claims. The remaining configuration is similar to that of the sheet-shaped covering 1 according to illustrative example 1 not falling under the scope of the claims. Therefore, a description thereof is omitted.

The transparent section 12 is a portion at least covering the insertion opening 102, but the position in the transparent section 12 that covers the insertion opening 102 may be determined and implemented appropriately by the user. For example, in this illustrative example not falling under the scope of the claims, when the transparent section 12 has a substantially rectangular shape in which one end is curved, and the substantially rectangular transparent section 12 is attached to the body surface so that the direction of the long sides thereof is substantially parallel to the extending direction of the vessel, then depending on the user, the portion of the transparent section 12 that actually covers the insertion opening 102 may be near the curved end, or the portion may be distant from the curved end, as illustrated in FIG. 9.

That is, by having a position in the transparent section 12 that is distant from the curved end cover the insertion opening 102 in the body surface, the portion of the tubular member 100 that extends into the body extends from the insertion opening 102 towards the curved end, and as illustrated in FIG. 9, the distal end 101 is positioned in an area covered by the transparent section 12. In other words, a position on the body surface near the distal end 101 of the tubular member 100 is also covered by the transparent section 12. Therefore, in this illustrative example not falling under the scope of the claims the temperature sensitive section 13 is disposed near the transparent section 12 in the orthogonal direction D so that leakage of the liquid medicine or the like outside of the vessel can be detected by the temperature sensitive section 13. This configuration allows an increase in the probability that leakage of the liquid medicine or the like outside of the vessel can be detected by the temperature sensitive section 13 even when the position of the body surface near the distal end 101 of the tubular member 100 is located within the area covered by the transparent section 12.

The distal end 101 of the tubular member 100 is still positioned near the vessel even when positioned outside of the vessel. Therefore, this configuration is better as the minimum distance between the transparent section 12 and the temperature sensitive section 13 in the orthogonal direction D is shorter, with the minimum distance preferably being 10 mm or less. In particular, the minimum distance in the orthogonal direction D between the portion of the body surface that is near the distal end 101 of the tubular member 100 and the temperature seisitive section 13 also changes depending on factors such as the width of the transparent section 12 in the orthogonal direction D and the position, in the orthogonal direction D, of the portion covering the insertion opening 102 within the entire transparent section 12. Hence, the minimum distance between the transparent section 12 and the temperature sensitive section 13 in the orthogonal direction D along the extending direction B of the sheet-shaped covering 11 is most preferably set to 0 mm to dispose the transparent section 12 and the temperature sensitive section 13 next to each other. Details on a configuration in which the transparent section 12 and the temperature sensitive section 13 are disposed next to each other in the orthogonal direction D are described below (for example, see FIG. 10).

In this illustrative example not falling under the scope of the claims the width of the transparent section 12 in the orthogonal direction D is set to 10 mm or greater in order to guarantee sufficient visibility of the insertion opening 102 and maintain handleability when the user attaches the sheet-shaped covering 11. The width of the transparent section 12 in the orthogonal direction D is preferably 30 mm or less so that the temperature sensitive section 13 can easily detect leakage of liquid medicine to the outside of the vessel from the distal end 101 of the tubular member 100 that is positioned near the vessel.

Furthermore, although a first temperature sensitive section 130 and a second temperature sensitive section 131 are provided on the sides of the transparent section 12 in the orthogonal direction D as the temperature sensitive section 13 in this illustrative example not falling under the scope of the claims a configuration may be adopted in which the temperature sensitive section 13 is provided on only one side of the transparent section 12. When providing the temperature sensitive section 13 on both sides of the transparent section 12 in the orthogonal direction D as in this illustrative example not falling under the scope of the claims however, even if the distal end 101 of the tubular member 100 is slightly separated from the vessel in the orthogonal direction D, leakage of the liquid medicine or the like can at least be detected by whichever of the first temperature sensitive section 130 and second temperature sensitive section 131 is closer to the distal end 101 of the tubular member 100. Therefore, as compared to when the temperature sensitive section 13 is provided on only one side, leakage of the liquid medicine or the like from the distal end 101 can be detected more reliably.

Furthermore, in this illustrative example not falling under the scope of the claims, the length of the temperature sensitive section 13 in a direction substantially parallel to the extending direction of the vessel (in the case of the temperature sensitive section 13 in FIG. 9, the length of the long sides of the first and second temperature sensitive sections 13) is set longer than the length of the transparent section 12 in the same direction, and the transparent section 12 and temperature sensitive section 13 are arranged so that the temperature sensitive section 13 is positioned in the orthogonal direction D with respect to any position of the transparent section 12. By adopting this arrangement, even if the portion of the transparent section 12 covering the insertion opening 102 differs by user, the temperature sensitive section 13 is always positioned in the orthogonal direction D with respect to the portion covering the insertion opening 102. Therefore, the probability of detecting leakage of liquid medicine or the like outside the vessel with the temperature sensitive section 13 is further increased.

In this illustrative example not falling under the scope of the claims when viewed from the thickness direction A, the shape of the transparent section 12 is substantially rectangular, and the shape of the temperature sensitive section 13 is rectangular. As long as the above-described positional relationship between the transparent section 12 and the temperature sensitive section 13 is satisfied, however, other shapes may be adopted. For example, the transparent section 12 and the temperature sensitive section 13 may be elliptical or may be an approximate polygonal shape other than a quadrilateral. In this illustrative example not falling under the scope of the claims the whole shape of the sheet-shaped covering 11 when viewed from the thickness direction A is a substantially quadrilateral shape, but the sheet-shaped covering 11 may be a different shape, such as a circle, an ellipse, or a polygon other than a quadrilateral.

illustrative example 3 not falling under the scope of the claims Next, with reference to FIG. 10, a sheet-shaped covering 21 according to illustrative example 3 not falling under the scope of the claims is described. FIG. 10 illustrates the sheet-shaped covering 21 in a state of being attachable to a body surface, as viewed from the thickness direction A of the sheet-shaped covering 21. The sheet-shaped covering 21 has a different configuration than the sheet-shaped covering 1 according to illustrative examples 1 and 2 not falling under the scope of the claims in that a temperature sensitive section 23 is positioned to surround at least a portion of a transparent section 22 in the extending direction B of the sheet-shaped covering 21, the transparent section 22 and the temperature sensitive section 23 are disposed adjacent to each other in the extending direction B of the sheet-shaped covering 21, and no extending section exists when viewed from the thickness direction A. For the remaining configuration, a similar configuration to the configuration already described in illustrative example 1 not falling under the scope of the claims is used. Therefore, a description is omitted.

Stating that "the temperature sensitive section is positioned to surround at least a portion of the transparent section in the extending direction of the sheet-shaped covering" refers to a positional relationship between the transparent section and the temperature sensitive section such that, when viewing the sheet-shaped covering from the thickness direction A, a line passing through the transparent section can be formed from two points in one continuous temperature sensitive section. For example, the dashed line in FIG. 10 is an imaginary line formed by connecting points X and Y in one continuous temperature sensitive section 23. This line passes through the transparent section 22. In other words, with the sheet-shaped covering 21 in this illustrative example not falling under the scope of the claims, such a line can be formed using two points in the temperature sensitive section in a plane when viewing the sheet-shaped covering from the thickness direction A. Therefore, the sheet-shaped covering 21 in this illustrative example not falling under the scope of the claims is configured so that "the temperature sensitive section 23 is positioned to surround at least a portion of the transparent section 22 in the extending direction B of the sheet-shaped covering 21".

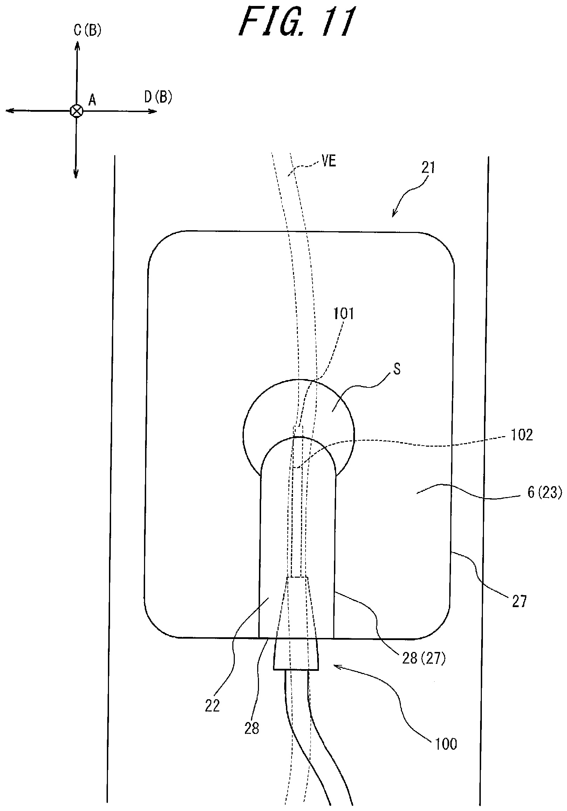

FIG. 11 illustrates a state in which the sheet-shaped covering 21 illustrated in FIG. 10 is attached to a body surface so as to cover a portion of a vessel in the body. The dashed lines in FIG. 11 represent a vessel VE in the body, specifically a venous blood vessel in the arm. FIG. 11 illustrates a state in which a portion of the distal end 101 of the tubular member 100 is positioned further outside than the outer wall of the vessel, and the liquid medicine from the distal end 101 has leaked outside of the vessel. In this illustrative example not falling under the scope of the claims, in a state in which the sheet-shaped covering 21 is attached to the body surface so as to cover a portion of the vessel, a direction C is parallel to the extending direction of the vessel among the extending directions B of the sheet-shaped covering 21, and the temperature sensitive section 23 is continuously disposed from a position in the direction C with respect to at least a portion of the transparent section 22 to a position in an orthogonal direction D, which is orthogonal to the direction C, with respect to at least the portion of the transparent section 22. Therefore, the probability that leakage of the liquid medicine or the like cannot be detected in the temperature sensitive section 23 depending on the position of the distal end 101 of the tubular member 100 can be reduced.

The area S in FIG. 11 indicates a portion of the display surface 6 of the temperature sensitive section 23 at which the color has changed due to the change in temperature of the body surface that occurred due to leakage of the liquid medicine or the like to the outside of the vessel VE. In this way, upon a change in temperature in the body surface, a change in color appears on the display surface 6, which is the surface within the temperature sensitive section 23 opposite the contact surface 5 that contacts the body surface (for example, see FIG. 3). Therefore, by the change in color of the temperature sensitive section 23, a health professional can confirm, from the outside, that liquid medicine or the like has leaked outside the vessel.

Furthermore, in this illustrative example not falling under the scope of the claims as illustrated in FIGS. 10 and 11, the transparent section 22 and the temperature sensitive section 23 are disposed adjacent to each other in the extending directions B of the sheet-shaped covering 21. In other words, when the sheet-shaped covering 21 is viewed from the thickness direction A, the outline 27 of the temperature sensitive section 23 has a portion in common with a portion of the outline 28 of the transparent section 22.

In greater detail, in FIG. 10, other than one side at the bottom, the entire outline 28 of the transparent section 22 is shared in common with a portion of the outline 27 of the temperature sensitive section 23. This configuration further increases the probability of being able to detect, with the temperature sensitive section 23, a change in temperature of the body surface occurring near the outline 27 of the transparent section 22 due to liquid medicine or the like leaking outside of the vessel VE as compared to a configuration in which there is a gap between the transparent section and the temperature sensitive section in the extending direction B of the sheet-shaped covering (the case of the minimum distance in the extending direction B of the sheet-shaped covering being greater than 0 mm). Furthermore, for example when the tubular member 100 is repeatedly inserted and removed, leakage of the liquid medicine or the like outside the vessel may occur from a hole, in the outer wall of the vessel, that has already formed near the insertion opening 102, even when the distal end 101 is placed within the vessel. In this illustrative example not falling under the scope of the claims, the temperature sensitive section 23 is disposed to be adjacent to and surround at least a portion of the transparent section 22. Therefore, even when such leakage of the liquid medicine or the like occurs, the probability of the temperature sensitive section 23 being able to detect the change in temperature of the body surface due to the leakage of the liquid medicine or the like increases.

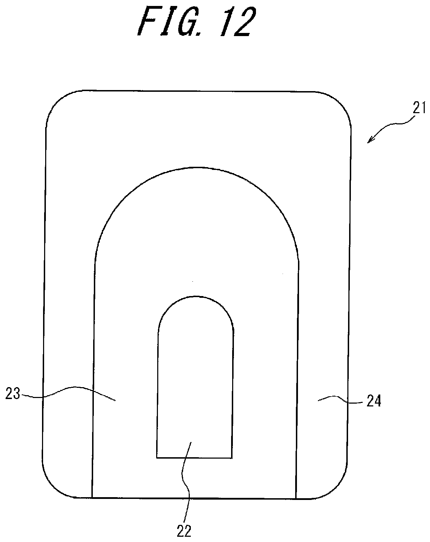

Here, when viewed from the thickness direction A, the temperature sensitive section 23 in this illustrative example not falling under the scope of the claims does not completely surround the transparent section 22, but as illustrated in FIG. 12, the temperature sensitive section 23 may be configured to completely surround the transparent section 22.

When viewed from the thickness direction A, the sheet-shaped covering 21 in this illustrative example not falling under the scope of the claims has no extending section 24, but rather is configured with the transparent section 22 and the temperature sensitive section 23. The configuration of the sheet-shaped covering is simplified by adopting this configuration. As illustrated in FIG. 12, however, an extending section 24 may be provided, and the extending section 24 may be configured to contact the body surface.

Furthermore, the shape of the transparent section 22 and the temperature sensitive section 23 when viewed from the thickness direction A is not limited to the substantially rectangular shape illustrated in this illustrative example not falling under the scope of the claims (specifically, the shape formed by one short side of a rectangle_{:} being an arc that protrudes outward). As long as the transparent section 22 and the temperature sensitive section 23 satisfy the above-described positional relationship, i.e. as long as the temperature sensitive section 23 is positioned to surround at least a portion of the transparent section 22 in the extending direction B of the sheet-shaped covering 21, any shape may be adopted.

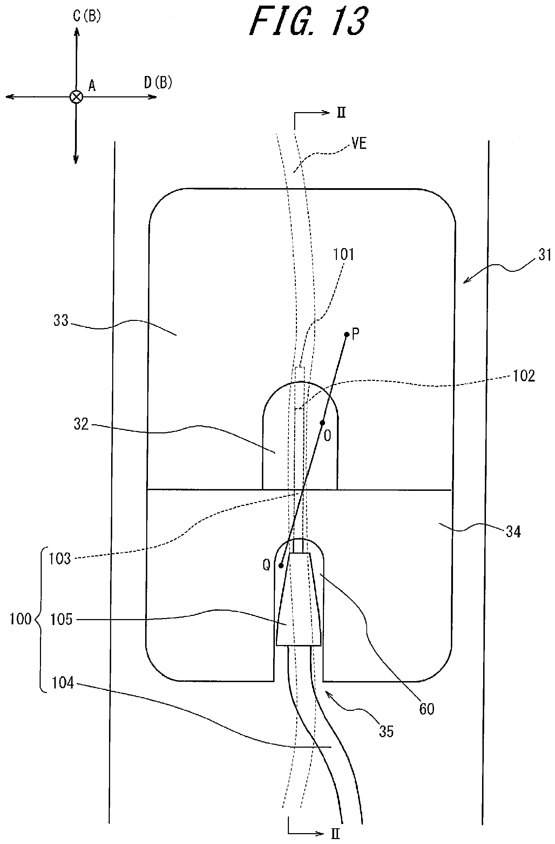

illustrative example 4 not falling under the scope of the claims Next, with reference to FIG. 13, a sheet-shaped covering 31 according to illustrative example 4 not falling under the scope of the claims is described. FIG. 13 illustrates the sheet-shaped covering 31 attached to a body surface so as to cover a portion of a vessel in the body, as viewed from the thickness direction A of the sheet-shaped covering 31. FIG. 14 illustrates the layered structure, in the thickness direction A, of the sheet-shaped covering 31 in a state before attachment to the body surface is possible, i.e. in a state in which the release layer or the like that is removed at the time of attachment to the body surface is provided on one surface ("state before use"). The dashed lines in FIG. 13 represent a vessel VE. FIG. 14 is a cross-section along the II-II line in FIG. 13 illustrating the sheet-shaped covering 31 in the state before use.

As illustrated in FIG. 13, the sheet-shaped covering 31 in this illustrative example not falling under the scope of the claims includes a transparent section 32, a temperature sensitive section 33, and an extending section 34. The sheet-shaped covering 31 in a state of being attachable to the body surface has a different configuration than the sheet-shaped covering 1 according to illustrative example 1 not falling under the scope of the claims 1 in that a notch 60 is provided at one end of the extending section 34, which is one end 35 of the sheet-shaped covering 31, the temperature sensitive section 33 is positioned to surround the transparent section 32 in the extending direction B of the sheet-shaped covering 31, and the transparent section 32 and the temperature sensitive section 33 are disposed adjacent to each other in the extending direction B of the sheet-shaped covering 31. For the remaining configuration of the sheet-shaped covering 31, the configuration already described in illustrative example not falling under the scope of the claims is used. Therefore, a detailed description is omitted. Furthermore, the configuration is the same as in illustrative example 3 not falling under the scope of the claims with regard to the temperature sensitive section 33 being positioned to surround the transparent section 32 in the extending direction B of the sheet-shaped covering 31 and with regard to the transparent section 32 and the temperature sensitive section 33 being disposed adjacent to each other in the extending direction B of the sheet-shaped covering 31. Therefore, a description of these points is omitted.

As illustrated in FIG. 13, a notch 60, which can contain part of the portion of the tubular member 100 that extends outside of the body, is provided at one end of the extending section 34 in this illustrative example not falling under the scope of the claims. With this configuration, since the extending section 34 includes an adhesive layer 34b, as illustrated in FIG. 14, movement with respect to the body surface of the portion of the tubular member 100 extending outside of the body is restricted by the portion of the extending section 34 surrounding the notch 60. As a result, the distal end 101 of the tubular member 100 is less likely to slip out of the vessel.

The transparent section 32, temperature sensitive section 33, and notch 60 of the extending section 34 are attached to the body surface so as to be aligned in the extending direction of the vessel. Therefore, in the sheet-shaped covering 31, the transparent section 32, temperature sensitive section 33, and notch 60 are disposed in a substantially straight line in the extending direction B. Stating that "the transparent section, temperature sensitive section, and notch are disposed in a substantially straight line in the extending direction of the sheet-shaped covering" means that there exists an imaginary line passing through each of the transparent section, temperature sensitive section, and notch when the sheet-shaped covering is viewed from the thickness direction. For example, the line illustrated in FIG. 13 is a line connecting point O in the transparent section 32, point P in the temperature sensitive section 33, and point Q in the notch 60. Since such points O, P, and Q exist, the sheet-shaped covering 31 illustrated in FIG. 13 is configured so that the transparent section 32, temperature sensitive section 33, and notch 60 are disposed in a substantially straight line in the extending direction B of the sheet-shaped covering 31.

When viewed from the thickness direction A, the notch 60 of this illustrative example not falling under the scope of the claims has an elongated, substantially rectangular shape so as to be able to contain a portion of the tubular member 100, but the notch 60 is not limited to this shape. For example, the notch 60 may be substantially triangular. The tubular member 100 illustrated in FIG. 13 includes an indwelling needle 103, a tube 104 extending outside of the body, and a connector 105 that connects one end of the indwelling needle 103 to one end of the tube 104 so that a hollow portion defined by the indwelling needle 103 and a hollow portion defined by the tube 104 are in communication. In this illustrative example not falling under the scope of the claims the connector 105 is contained in the notch 60. Within the tube 104, the opposite end from the end that is connected to the connector 105 (the proximal end) is connected to a bag of the liquid medicine or nutrients being administered.

Furthermore, while the notch 60 in this illustrative example not falling under the scope of the claims is provided at one end of the extending section 34, for example a configuration may be adopted in which the transparent section 32 extends to one end 35 of the sheet-shaped covering 31, with the notch 60 being provided at one end of the transparent section 32.

Next, with reference to FIG. 14, the sheet-shaped covering 31 in the state before use is described. FIG. 14 only illustrates the relationship between the layers in the sheet-shaped covering 31 in the state before use and does not represent the actual thickness of each layer. The actual thickness of each layer may be set appropriately in accordance with purpose or use.

As illustrated in FIG. 14, the transparent section 32 includes a resin layer 32a and an adhesive layer 32b. The temperature sensitive section 33 includes an adhesive layer 33d, a black layer 33c, a temperature sensitive layer 33b, a substrate layer 33a, and a surface layer 33e. Furthermore, the extending section 34 includes a resin layer 34a and the adhesive layer 34b, A connector 38, which is a transparent resin sheet, constitutes the resin layer 32a of the transparent section 32, the surface layer 33e of the temperature sensitive section 33, and the resin layer 34a of the extending section 34. The specific configuration of these components and layers is similar to the configuration described in illustrative example not falling under the scope of the claims.

The sheet-shaped covering 31 in the state before use is provided with the release layer 61 on the surface, in the thickness direction A, that attaches to the body surface. The release layer 61 for example covers the adhesive layer 32b of the transparent section 32, the adhesive layer 33d of the temperature sensitive section 33, and the adhesive layer 34b of the extending section 34. When the sheet-shaped covering 31 is actually used, the release layer 61 is removed by the user. By removal of the release layer 61, the adhesive layer 32b of the transparent section 32, the adhesive layer 33d of the temperature sensitive section 33, the adhesive layer 34b of the extending section 34, and the like are exposed, allowing the sheet-shaped covering 31 to be adhered to the body surface. The release layer 61 is a resin sheet and may be either transparent or opaque.

A configuration may be adopted in which a separate release layer (second release layer) covering at least the display surface 6 of the temperature sensitive section 33 is provided on the opposite side from the release layer 61. This configuration can reduce the likelihood of the surface of the temperature sensitive section 33 being damaged before use.

illustrative example 5 not falling under the scope of the claims Next, a sheet-shaped covering 41 according to illustrative example 5 not falling under the scope of the claims described. The sheet-shaped covering 41 includes a transparent section 42, a temperature sensitive section 43, and an extending section 44. As compared to the sheet-shaped covering 1 according to illustrative example 1 not falling under the scope of the claims the connector in the sheet-shaped covering 41 has a different configuration. The remaining configuration is the same as the configuration already described in the above illustrative examples not falling under the scope of the claims Therefore, a description is omitted.

FIG. 15 illustrates the sheet-shaped covering 41 in a state of being attachable to the body surface as viewed from the thickness direction A, and FIGS. 16(a) and 16(b) are cross-sectional diagrams of the sheet-shaped covering 41 respectively along the III-III and IV-IV lines in FIG. 15.

As illustrated in FIG. 15, the extending section 44 in this illustrative example 1 not falling under the scope of the claims includes a first extending section 440 positioned to surround the transparent section 42 and a second extending section 441 positioned to surround the temperature sensitive section 43 and is provided with the connector 803 that connects the first extending section 440 and the second extending section 441 by being connected to one surface of the first extending section 440 and to one surface of the second extending section 441. This means that when the connector 803 does not exist, the first extending section 440 and the second extending section 441 are two separate, unconnected extending sections.

As illustrated in FIGS. 16(a) and 16(b), the transparent section 42 and the first extending section 440 are connected by a common first connector 801, and the temperature sensitive section 43 and the second extending section 441 are connected by a common second connector 802. The first connector 801 constitutes one layer in each of the transparent section 42 and the first extending section 440. The second connector 802 constitutes one layer in each of the temperature sensitive section 43 and the second extending section 441. The reference numeral 44b in FIGS. 16(a) and 16(b) represents the adhesive layer in the extending section 44, whereas the reference numerals 43b and 43c represent the temperature sensitive layer and the black layer of the temperature sensitive section 43. The specific configuration of these layers is similar to that of the adhesive layer 4b of the extending section 4, the temperature sensitive layer 3b of the temperature sensitive section 3, and the black layer 3c of the temperature sensitive section 3 in illustrative example 1 not falling under the scope of the claims

In other words, the connector 803 is a third connector 803 that connects the first connector 801 and the second connector 802 by being layered onto the first connector 801 in a portion of the first extending section 440 and being layered onto the second connector 802 in a portion of the second extending section 441.

In this way, in this illustrative example not falling under the scope of the claims the first connector 801, second connector 802, and third connector 803 constitute a composite connector 48. As a whole, the composite connector 48 connects the transparent section 42 and the temperature sensitive section 43. The first through third connectors 801 to 803 are each a transparent resin sheet.

The connector 48 is not limited to the above-described configuration. For example, since the third connector 803 is a transparent resin sheet, the third connector 803 may be layered onto the transparent section 42 and/or the temperature sensitive section 43 to constitute one layer of the transparent section 42 and/or the temperature sensitive section 43.

### <Embodiment 1>

Next, a sheet-shaped covering 51 according to Embodiment 1 is described. The sheet-shaped covering 51 includes a transparent section 52, a temperature sensitive section 53, and an extending section 54. As compared to the sheet-shaped covering 1 according to illustrative example 1 not falling under the scope of the claims, mainly the layered configuration of the temperature sensitive section and the configuration of the connector differ in the sheet-shaped covering 51. The remaining configuration is the same as the configuration already described in the above illustrative examples not falling under the scope of the claims Therefore, a description is omitted.

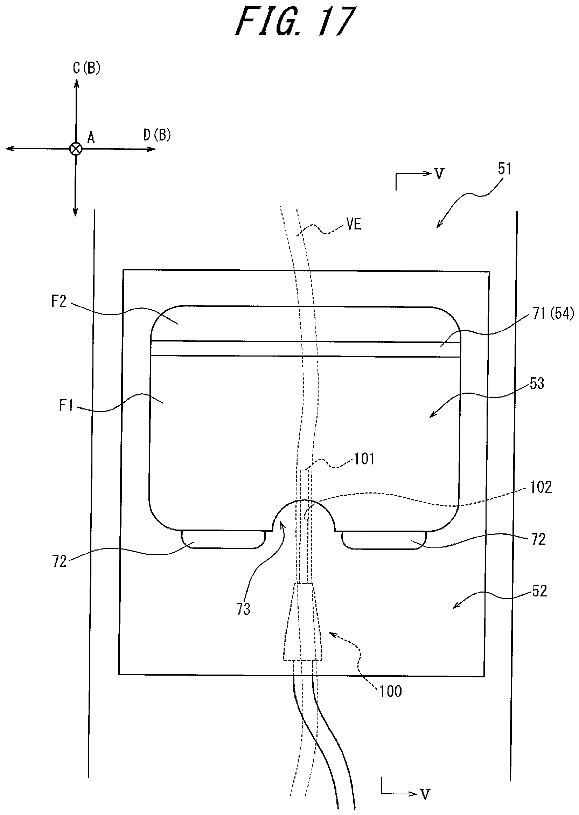

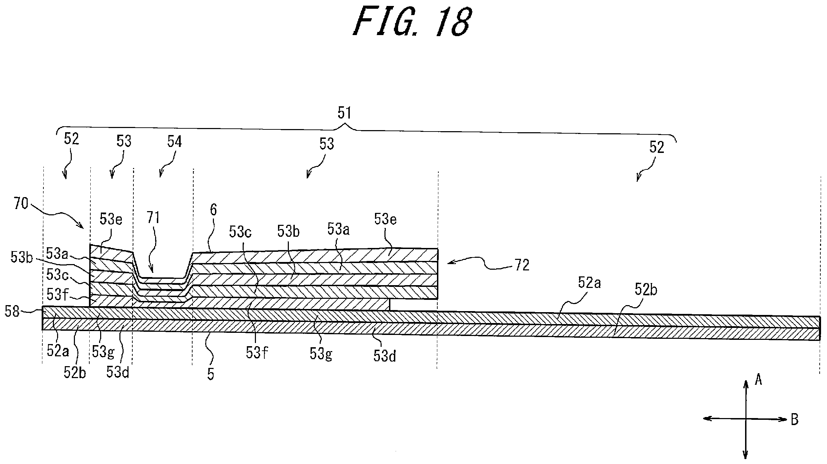

FIG. 17 illustrates the sheet-shaped covering 51, as viewed from the thickness direction A, in a state of attachment to the body surface so as to cover a portion of a vessel in the body. FIG. 18 is a cross-sectional diagram of the sheet-shaped covering 51 along the V-V line in FIG. 17. FIG. 18 illustrates the relationship between the layers at each position but does not represent the actual thickness of each layer at each position. The actual thickness of each layer may be set appropriately in accordance with purpose or use.

As illustrated in FIG. 18, in the sheet-shaped covering 51, the connector 58 connecting the transparent section 52 and the temperature sensitive section 53 is a transparent resin sheet. As illustrated in FIGS. 17 and 18, this resin sheet that serves as the connector 58 constitutes one layer of each of the temperature sensitive section 53 and the transparent section 52 in the thickness direction A of the sheet-shaped covering 51.

The temperature sensitive section 53 includes the transparent resin sheet that serves as the connector 58 and a temperature sensitive sheet 70 that changes color in accordance with temperature and is layered onto the resin sheet at the display surface 6 side of the temperature sensitive section 53, at the opposite side from the contact surface 5 that contacts the body surface.

In greater detail, the temperature sensitive section 53 of this embodiment includes the following layers in this order from the display surface 6 side: a surface layer 53e, a substrate layer 53a, a temperature sensitive layer 53b, a black layer 53c, a first adhesive layer 53f, a resin layer 53g, and a second adhesive layer 53d. The surface layer 53e, substrate layer 53a, temperature sensitive layer 53b, black layer 53c, and first adhesive layer 53f are constituted by the temperature sensitive sheet 70, whereas the resin layer 53g is constituted by the transparent resin sheet. The first adhesive layer 53f is a layer that directly contacts and adheres to the resin sheet, which is one constituent element, thereby adhering the temperature sensitive sheet 70 as an integrated constituent element to the resin sheet. The second adhesive layer 53d is a transparent layer that, by directly contacting and adhering to the body surface, adheres the entire sheet-shaped covering 51, which includes the temperature sensitive sheet 70 and the resin sheet, to the body surface.

In this way, on the surface facing the resin sheet that serves as the connector 58, the temperature sensitive sheet 70 is provided with the first adhesive layer 53f that serves as an adhesive section adhering to the resin sheet. On the surface at the body surface side of the resin sheet that serves as the connector 58, the second adhesive layer 53d is provided as an adhesive section that can adhere to the body surface. For the sake of convenience, the adhesive section configured with the first adhesive layer 53f of the temperature sensitive sheet 70 is referred to as the "first adhesive section", and the adhesive section configured with the second adhesive layer 53d layered onto the resin sheet that serves as the connector 58 is referred to as the "second adhesive section".

The transparent section 52 includes a resin layer 52a and an adhesive layer 52b. The resin layer 52a is configured with the above-described transparent resin sheet that serves as the connector 58. The adhesive layer 52b in this embodiment is integrated with the second adhesive layer 53d of the temperature sensitive section 53, but these adhesive layers may be configured as separate adhesive layers.

In this embodiment, the adhesive force of the first adhesive layer 53f, which serves as the first adhesive section, with respect to the resin sheet is smaller than the adhesive force of the second adhesive layer 53d, which serves as the second adhesive section, with respect to the body surface. Therefore, when the sheet-shaped covering 51 is in a state of attachment to the body surface via the second adhesive section, the temperature sensitive sheet 70 can easily be peeled off the resin sheet that serves as the connector 58. By setting the relative magnitudes of the adhesive forces in this way, the transparent area (transparent section 52) when viewed from the thickness direction A can be extended by peeling the temperature sensitive sheet 70 off the resin sheet as necessary, making it easier to visually confirm the position of the insertion opening 102 for the tubular member 100 in the body surface. Such relative magnitudes of the adhesive forces can, for example, be implemented by varying the types of adhesives that are used.

In this embodiment, the first adhesive layer 53f that serves as the first adhesive section is a pressure-sensitive adhesive layer (a removable adhesive) that is adhesive enough to adhere the temperature sensitive sheet 70 to and separate the temperature sensitive sheet 70 from the resin sheet that serves as the connector 58 at least a plurality of times. With this configuration, after the temperature sensitive sheet 70 is peeled off the resin sheet and the position of the insertion opening 102 for the tubular member 100 or the position of the distal end 101 of the tubular member 100 is confirmed, the temperature sensitive sheet 70 can once again be adhered to an appropriate position. Note that "pressure-sensitive adhesive" is one form of adhesion.

In this embodiment, the second adhesive layer 53d that serves as the second adhesive section is also a pressure-sensitive adhesive layer that is adhesive enough to adhere the sheet-shaped covering 51 to and separate the sheet-shaped covering 51 from the body surface at least a plurality of times. The pressure-sensitive adhesive layers forming the first adhesive layer 53f and the second adhesive layer 53d may for example be configured using a silicon adhesive such as a silicon gel adhesive, a rubber adhesive, an acrylic adhesive, or the like.

Furthermore, in this embodiment, only a portion of the temperature sensitive sheet 70 is configured for adhesion to and removal from the resin sheet, which serves as the connector 58, a plurality of times. Specifically, as illustrated in FIG. 17 and 18, the temperature sensitive sheet 70 includes a band-shaped fixed section 71 that is fixed to the resin sheet by contact bonding, heat sealing, or the like. The portion of the temperature sensitive sheet 70 other than the fixed section 71 can be adhered to and peeled off the resin sheet. By the temperature sensitive sheet 70 including the fixed section 71, the position of the temperature sensitive sheet 70 with respect to the resin sheet when viewed from the thickness direction A can be prevented from moving each time the temperature sensitive sheet 70 is adhered to the resin sheet, thereby making it less troublesome for the user to adhere and peel off the temperature sensitive sheet 70. As illustrated in FIG. 18, in order to fix the fixed section 71 of the temperature sensitive sheet 70 to the resin sheet, the fixed section 71 is pressed towards the resin sheet to contract in the thickness direction A. Depending on the fixing method, however, a fixed section that is not in a contracted state can be formed.

As illustrated in FIG. 17, when the sheet-shaped covering 51 is viewed from the thickness direction A, the fixed section 71 in this embodiment is formed as a band that divides the temperature sensitive section 53 into two areas and that does not present a change in color in accordance with temperature. Only the portion of the temperature sensitive sheet 70 corresponding to the area F1 can be adhered to and removed from the resin sheet, whereas the portion of the temperature sensitive sheet 70 corresponding to the other area F2 is continuously adhered to the resin sheet. In this way, by providing a portion in the temperature sensitive sheet 70 that is continuously adhered to the resin sheet, this portion can be used as a color indicator, and a comparison with the color of the color indicator that is in a state of continuous adhesion to the resin sheet makes it easy to determine any variation in the degree of temperature sensitivity based on a reduction in the adhesion strength of the first adhesive layer 53f due to repeated adhesion and separation or based on the occurrence of a gap between the temperature sensitive sheet 70 and the resin sheet, which may occur upon re-adhering the temperature sensitive sheet 70 after separation.

The fixed section 71 of this embodiment is a shaped as a straight band that divides the temperature sensitive section 53 into a plurality of areas when viewed from the thickness direction A, but the fixed section 71 is not limited to being a straight band and may, for example, be a curved band. The number of areas into which the fixed section divides the temperature sensitive section is also not limited to two. The temperature sensitive section may be divided into three or more areas. Furthermore, when viewed from the thickness direction A in a state of attachment to the body surface (see FIG. 17), the fixed section 71 in this embodiment is a band that extends in a direction D orthogonal to a direction C that is substantially parallel to the extending direction of the vessel and divides the temperature sensitive section 53 into two areas in the direction C that is substantially parallel to the extending direction of the vessel. For example, however, the fixed section 71 may be a band that extends in the direction C that is substantially parallel to the extending direction of the vessel and divides the temperature sensitive section 53 into two areas in the direction D orthogonal to the direction C that is substantially parallel to the extending direction of the vessel.

Furthermore, when viewed from the thickness direction A of the sheet-shaped covering 51, the fixed section 71 in this embodiment constitutes an extending section 54 that does not present a change in color in accordance with temperature, but the fixed section may be a portion of the temperature sensitive section that presents a change in color. In this case, since the fixed section is continually adhered to the resin sheet, the fixed section may be used as the above-described color indicator.

In this embodiment, the first adhesive layer 53f serving as the first adhesive section is provided on almost the entire surface of the temperature sensitive sheet 70 on the resin sheet side (the entire surface on the resin sheet side excluding the surface of the below-described grip 72), but a configuration may be adopted in which the first adhesive section is provided only in a smaller area of the temperature sensitive sheet 70 on the resin sheet side. In this case, in addition to or instead of using different adhesives for the first adhesive section and the second adhesive section, the area of the first adhesive section per unit area when viewing the sheet-shaped covering 51 from the thickness direction is preferably set smaller than the area of the second adhesive section per unit, so that the adhesive force of the first adhesive section with respect to the resin sheet is smaller than the adhesive force of the second adhesive section with respect to the body surface.

As illustrated in FIGS. 17 and 18, the temperature sensitive sheet 70 in this embodiment includes a grip 72 that can be gripped when peeling the first adhesive layer 53f, which serves as the first adhesive section, off the resin sheet, which serves as the connector 58. In greater detail, the grip 72 in the temperature sensitive sheet 70 of this embodiment is configured by one end, in the extending direction B of the sheet-shaped covering 51, where the first adhesive layer 53f is not provided. Providing such a grip 72 improves handleability when removing the temperature sensitive sheet 70 from the resin sheet. The grip 72 in this embodiment is formed at one end of the temperature sensitive section 53 in a direction C that is substantially parallel to the extending direction of the vessel, as illustrated in FIG. 17. For example, however, the grip 72 may be formed at a different end in a direction D orthogonal to the direction C that is substantially parallel to the extending direction of the vessel.

As illustrated in FIG. 17, a notch 73 that serves as a guide for the user to appropriately position the sheet-shaped covering 51 with respect to the insertion opening 102 for the tubular member 100 in the body surface is formed at one end of the temperature sensitive section 53 in this embodiment.

A sheet-shaped covering according to this disclosure may thus be implemented with a variety of specific configurations and is not limited to the configurations in the above-described embodiments. In particular, combining the configurations described in the embodiments to yield a new sheet-shaped covering is within the technical scope of this disclosure. For example, replacing the various layered structures in the thickness direction A of the sheet-shaped covering 1 described in illustrative exmaple 1 not falling under the scope of the claims with the layered structures used in the sheet-shaped coverings 11, 12, and 31 in illustrative example 2 not falling under the scope of the claims to 4 is within the technical scope of this disclosure, as is adopting the notch 60 described in illustrative example 4 not falling under the scope of the claims in the sheet-shaped coverings 1, 11, and 21 in illustrative examples 1-3 not falling under the scope of the claims.

In the above-described embodiments, except where otherwise noted, adjacent layers may be connected by a variety of methods such as a method for adhesion with a separately provided layer or the like of transparent adhesive, a method for fusing layers to each other, or the like.

### INDUSTRIAL APPLICABILITY

This disclosure relates to a sheet-shaped covering for medical use.

### REFERENCE SIGNS LIST

1, 11, 21, 31, 41, 51 Sheet-shaped covering
2, 2', 12, 22, 32, 42, 52 Transparent section
2a, 32a, 52a Resin layer of transparent section
2b, 32b, 52b Adhesive layer of transparent section
3, 3', 3", 13, 23, 33, 43, 53 Temperature sensitive section
3a, 3a', 3a", 33a, 53a Substrate layer of temperature sensitive section
3b, 3b', 33b, 43b, 53b Temperature sensitive layer of temperature sensitive section
3c, 3c', 3c", 33c, 43c, 53c Black layer of temperature sensitive section
3d, 3d', 33d Adhesive layer of temperature sensitive section
3e, 3e', 3e", 33e, 53e Surface layer of temperature sensitive section
4, 14, 24, 34, 44, 54 Extending section
4a, 34a Resin layer of extending section
4b, 34b, 44b Adhesive layer of extending section
5 Contact surface of temperature sensitive section
6 Display surface of temperature sensitive section
7 Adhesive section
8, 38, 48, 58 Connector
9 Transparent adhesive section
27 Outline of temperature sensitive section
28 Outline of transparent section
53d First adhesive layer of temperature sensitive section (second adhesive section)
53f Second adhesive layer of temperature sensitive section (first adhesive section)
53g Resin layer of temperature sensitive section
60 Notch
61 Release layer
70 Temperature sensitive sheet
71 Fixed section
72 Grip
73 Notch
100 Tubular member
101 Distal end of tubular member
102 Insertion opening
103 Indwelling needle
104 Tube
105 Connector
130 First temperature sensitive section
131 Second temperature sensitive section
440 First extending section
441 Second extending section
801 First connector
802 Second connector
803 Third connector
BS Body surface
VE Vessel
LM Liquid medicine or the like leaking outside of vessel
A Thickness direction of sheet-shaped covering
B Extending direction of sheet-shaped covering
C Direction parallel to extending direction of vessel
D Direction orthogonal to extending direction of vessel
F1 One area of the temperature sensitive section divided by the fixed section
F2 Another area of the temperature sensitive section divided by the fixed section
O Point in the transparent section when the sheet-shaped covering is viewed from the thickness direction
P Point in the temperature sensitive section when the sheet-shaped covering is viewed from the thickness direction
Q Point in the notch when the sheet-shaped covering is viewed from the thickness direction
X, Y Points in the temperature sensitive section when the sheet-shaped covering is viewed from the thickness direction
Lmin Minimum distance between transparent section and temperature sensitive section

## Claims

1. A sheet-shaped covering (1, 51) to be attached to a body surface (BS), the sheet-shaped covering (1, 51) comprising:
a transparent section (2, 52) configured to cover an insertion opening (102) in the body surface (BS) for a tubular member (100) to be inserted into a body through the body surface (BS) so that a distal end (101) of the tubular member (100) may be placed inside the body;
a temperature sensitive section (3, 53) that is positioned near the transparent section (2, 52) in the extending direction (B) of the sheep-shaped covering (1) in the plane of the sheet when viewing the sheet-shaped covering (1) from a thickness direction (A), which is configured to contact the body surface (BS), and changes color in accordance with temperature of the body surface (BS); and
a transparent resin sheet connecting the transparent section (2, 52) and the temperature sensitive section (3, 53),
**characterized in that** the resin sheet forms one layer, in a thickness direction (A) of the sheet-shaped covering (1, 51), of each of the temperature sensitive section (3, 53) and the transparent section (2, 52), and
the temperature sensitive section (3, 53) includes the resin sheet and a temperature sensitive sheet (70) that changes color in accordance with temperature and is layered onto a side of a display surface (6) of the temperature sensitive section (3, 53) with respect to the resin sheet, the display surface (6) positioned at an opposite side from a contact surface (5) that is configured to contact the body surface (BS) with respect to the resin sheet.

2. The sheet-shaped covering (1, 51) of claim 1, wherein when the sheet-shaped covering (1, 51) is attached to the body surface (BS) so as to cover a portion of a vessel (VE) in the body, the temperature sensitive section (3, 53) is positioned in a direction (C) parallel to an extending direction of the vessel (VE) with respect to a portion of the transparent section (2, 52) covering the insertion opening (102).

3. The sheet-shaped covering (1, 51) of claim 1 or 2, wherein when the sheet-shaped covering (1, 51) is attached to the body surface (BS) so as to cover a portion of a vessel (VE) in the body, the temperature sensitive section (3, 53) is positioned in a direction (D) orthogonal to an extending direction of the vessel (VE) with respect to a portion of the transparent section (2, 52) covering the insertion opening.

4. The sheet-shaped covering (1, 51) of any one of claims 1 to 3, wherein the temperature sensitive section (3, 53) is positioned to surround at least a portion of the transparent section (2, 52).

5. The sheet-shaped covering (1, 51) of any one of claims 1 to 4, wherein when viewing the sheet-shaped covering (1, 51) from a thickness direction (A), an outline (27) of the temperature sensitive section (3, 53) has at least a portion in common with a portion or all of an outline (28) of the transparent section (2, 52).

6. The sheet-shaped covering (1, 51) of any one of claims 1 to 5, wherein the temperature sensitive section (3, 53) includes an adhesive section (7, 3d, 53d) on the contact surface (5) of the temperature sensitive section (3, 53) that contacts the body surface (BS), and the adhesive section (7, 3d, 53d) is capable of adhering to the body surface (BS).

7. The sheet-shaped covering (1, 51) of any one of claims 1 to 6, wherein the transparent section (2, 52) includes a transparent adhesive section (9, 2b, 52b) on a surface of the transparent section (2, 52) that faces the body surface (BS) when the sheet-shaped covering (1, 51) is attached to the body surface (BS).

8. The sheet-shaped covering (1, 51) of any one of claims 1 to 7, wherein the temperature sensitive sheet (70) includes, on a surface of the temperature sensitive sheet (70) facing the resin sheet, an adhesive section (53f) that adheres to the resin sheet.

9. The sheet-shaped covering (1, 51) of claim 8, wherein the temperature sensitive sheet (70) includes a grip (72) capable of being gripped when peeling the adhesive section (53f) off the resin sheet.

10. The sheet-shaped covering (1, 51) of claim 8 or 9, wherein
the adhesive section (53f) of the temperature sensitive sheet (70) is designated as a first adhesive section, and a second adhesive section (53d) capable of adhering to the body surface (BS) is disposed on the resin sheet, and
an adhesive force of the first adhesive section (53f) with respect to the resin sheet is smaller than an adhesive force of the second adhesive section (53d) with respect to the body surface (BS).

## Patentansprüche

1. Blattförmige Abdeckung (1, 51), die an einer Körperoberfläche (BS) anzubringen ist, wobei die blattförmige Abdeckung (1, 51) umfasst:
einen transparenten Abschnitt (2, 52), der so konfiguriert ist, dass er eine Einführungsöffnung (102) in der Körperoberfläche (BS) für ein röhrenförmiges Element (100), das durch die Körperoberfläche (BS) in einen Körper eingeführt werden soll, abdeckt, sodass ein distales Ende (101) des röhrenförmigen Elements (100) im Inneren des Körpers angeordnet werden kann;
einen temperaturempfindlichen Abschnitt (3, 53), der in der Nähe des transparenten Abschnitts (2, 52) in der Erstreckungsrichtung (B) der blattförmigen Abdeckung (1) in der Ebene des Blattes angeordnet ist, wenn die blattförmige Abdeckung (1) aus einer Dickenrichtung (A) betrachtet wird, welcher so konfiguriert ist, dass er mit der Körperoberfläche (BS) in Kontakt tritt, und der seine Farbe in Übereinstimmung mit der Temperatur der Körperoberfläche (BS) ändert; und
eine transparente Harzfolie, die den transparenten Abschnitt (2, 52) und den temperaturempfindlichen Abschnitt (3, 53) verbindet,
**dadurch gekennzeichnet, dass** die Harzfolie in einer Dickenrichtung (A) der blattförmigen Abdeckung (1, 51) jeweils eine Schicht des temperaturempfindlichen Abschnitts (3, 53) und des transparenten Abschnitts (2, 52) bildet, und
der temperaturempfindliche Abschnitt (3, 53) die Harzfolie und eine temperaturempfindliche Folie (70) umfasst, die ihre Farbe in Übereinstimmung mit der Temperatur ändert und auf eine Seite einer Anzeigefläche (6) des temperaturempfindlichen Abschnitts (3, 53) in Bezug auf die Harzfolie geschichtet ist, wobei die Anzeigefläche (6) an einer gegenüberliegenden Seite von einer Kontaktfläche (5) positioniert ist, die so konfiguriert ist, dass sie mit der Körperoberfläche (BS) in Bezug auf die Harzfolie in Kontakt tritt.

2. Blattförmige Abdeckung (1, 51) nach Anspruch 1, wobei, wenn die blattförmige Abdeckung (1, 51) an der Körperoberfläche (BS) so angebracht ist, dass sie einen Abschnitt eines Gefäßes (VE) in dem Körper bedeckt, der temperaturempfindliche Abschnitt (3, 53) in einer Richtung (C) parallel zu einer Erstreckungsrichtung des Gefäßes (VE) in Bezug auf einen Teil des transparenten Abschnitts (2, 52), der die Einführungsöffnung (102) bedeckt, positioniert ist.

3. Blattförmige Abdeckung (1, 51) nach Anspruch 1 oder 2, wobei, wenn die blattförmige Abdeckung (1, 51) an der Körperoberfläche (BS) so angebracht ist, dass sie einen Abschnitt eines Gefäßes (VE) in dem Körper bedeckt, der temperaturempfindliche Abschnitt (3, 53) in einer Richtung (D) orthogonal zu einer Erstreckungsrichtung des Gefäßes (VE) in Bezug auf einen Teil des transparenten Abschnitts (2, 52), der die Einführungsöffnung bedeckt, positioniert ist.

4. Blattförmige Abdeckung (1, 51) nach einem der Ansprüche 1 bis 3, wobei der temperaturempfindliche Abschnitt (3, 53) so angeordnet ist, dass er mindestens einen Teil des transparenten Abschnitts (2, 52) umgibt.

5. Blattförmige Abdeckung (1, 51) nach einem der Ansprüche 1 bis 4, wobei, wenn die blattförmige Abdeckung (1, 51) aus einer Dickenrichtung (A) betrachtet wird, ein Umriss (27) des temperaturempfindlichen Abschnitts (3, 53) mindestens einen Teil mit einem Teil oder der Gesamtheit eines Umrisses (28) des transparenten Abschnitts (2, 52) gemeinsam hat.

6. Blattförmige Abdeckung (1, 51) nach einem der Ansprüche 1 bis 5, wobei der temperaturempfindliche Abschnitt (3, 53) einen Klebeabschnitt (7, 3d, 53d) auf der Kontaktfläche (5) des temperaturempfindlichen Abschnitts (3, 53) aufweist, der mit der Körperoberfläche (BS) in Kontakt tritt, und der Klebeabschnitt (7, 3d, 53d) in der Lage ist, an der Körperoberfläche (BS) zu haften.

7. Blattförmige Abdeckung (1, 51) nach einem der Ansprüche 1 bis 6, wobei der temperaturempfindliche Abschnitt (2, 52) einen transparenten Klebeabschnitt (9, 2b, 52b) auf einer Oberfläche des transparenten Abschnitts (2, 52) aufweist, die der Körperoberfläche (BS) zugewandt ist, wenn die blattförmige Abdeckung (1, 51) an der Körperoberfläche (BS) angebracht ist.

8. Blattförmige Abdeckung (1, 51) nach einem der Ansprüche 1 bis 7, wobei die temperaturempfindliche Folie (70) auf einer Oberfläche der temperaturempfindlichen Folie (70), die der Harzfolie zugewandt ist, einen Klebeabschnitt (53f) aufweist, der an der Harzfolie haftet.

9. Blattförmige Abdeckung (1, 51) nach Anspruch 8, wobei die temperaturempfindliche Folie (70) einen Griff (72) aufweist, der beim Ablösen des Klebeabschnitts (53f) von der Harzfolie gegriffen werden kann.

10. Blattförmige Abdeckung (1, 51) nach Anspruch 8 oder 9, wobei
der Klebeabschnitt (53f) der temperaturempfindlichen Folie (70) als ein erster Klebeabschnitt bezeichnet wird und ein zweiter Klebeabschnitt (53d), der in der Lage ist, an der Körperoberfläche (BS) zu haften, auf der Harzfolie angeordnet ist, und
eine Haftkraft des ersten Klebeabschnitts (53f) in Bezug auf die Harzfolie kleiner ist als eine Haftkraft des zweiten Klebeabschnitts (53d) in Bezug auf die Körperoberfläche (BS).

## Revendications

1. Élément de couverture en forme de feuille (1, 51) à fixer à une surface corporelle (BS), l'élément de couverture en forme de feuille (1, 51) comprenant :
une section transparente (2, 52) configurée pour couvrir une ouverture d'insertion (102) dans la surface corporelle (BS) pour l'insertion d'un élément tubulaire (100) à l'intérieur d'un corps à travers la surface corporelle (BS) de sorte qu'une extrémité distale (101) de l'élément tubulaire (100) puisse être placée à l'intérieur du corps ;
une section thermosensible (3, 53) qui est positionnée à proximité de la section transparente (2, 52) dans la direction d'extension (B) de l'élément de couverture en forme de feuille (1) dans le plan de la feuille lors de la visualisation de l'élément de couverture en forme de feuille (1) depuis une direction de l'épaisseur (A), qui est configurée pour venir au contact de la surface corporelle (BS), et change de couleur en fonction de la température de la surface corporelle (BS) ; et
une feuille de résine transparente reliant la section transparente (2, 52) et la section thermosensible (3, 53), **caractérisée en ce que** la feuille de résine forme une couche, dans une direction de l'épaisseur (A) de l'élément de couverture en forme de feuille (1, 51), de chacune de la section thermosensible (3, 53) et de la section transparente (2, 52), et
la section thermosensible (3, 53) comporte la feuille de résine et une feuille thermosensible (70) qui change de couleur conformément à la température et est stratifiée sur un côté d'une surface d'affichage (6) de la section thermosensible (3, 53) par rapport à la feuille de résine, la surface d'affichage (6) positionnée au niveau d'un côté opposé à une surface de contact (5) qui est configurée pour venir au contact de la surface corporelle (BS) par rapport à la feuille de résine.

2. Élément de couverture en forme de feuille (1, 51) selon la revendication 1, dans lequel lorsque l'élément de couverture en forme de feuille (1, 51) est fixé à la surface corporelle (BS) pour couvrir une partie d'un vaisseau (VE) dans le corps, la section thermosensible (3, 53) est positionnée dans une direction (C) parallèle à une direction d'extension du vaisseau (VE) par rapport à une partie de la section transparente (2, 52) couvrant l'ouverture d'insertion (102).

3. Élément de couverture en forme de feuille (1, 51) selon la revendication 1 ou 2, dans lequel l'élément de couverture en forme de feuille (1, 51) est fixé à la surface corporelle (BS) pour couvrir une partie d'un vaisseau (VE) dans le corps, la section thermosensible (3, 53) est positionnée dans une direction (D) orthogonale à une direction d'extension du vaisseau (VE) par rapport à une partie de la section transparente (2, 52) couvrant l'ouverture d'insertion.

4. Élément de couverture en forme de feuille (1, 51) selon l'une quelconque des revendications 1 à 3, dans lequel la section thermosensible (3, 53) est positionnée pour entourer au moins une partie de la section transparente (2, 52).

5. Élément de couverture en forme de feuille (1, 51) selon l'une quelconque des revendications 1 à 4, dans lequel lors de la visualisation de l'élément de couverture en forme de feuille (1, 51) depuis une direction de l'épaisseur (A), un contour (27) de la section thermosensible (3, 53) a au moins une partie en commun avec une partie ou la totalité d'un contour (28) de la section transparente (2, 52).

6. Élément de couverture en forme de feuille (1, 51) selon l'une quelconque des revendications 1 à 5, dans lequel la section thermosensible (3, 53) comporte une section adhésive (7, 3d, 53d) sur la surface de contact (5) de la section thermosensible (3, 53) qui vient au contact de la surface corporelle (BS), et la section adhésive (7, 3d, 53d) est capable d'adhérer à la surface corporelle (BS).

7. Élément de couverture en forme de feuille (1, 51) selon l'une quelconque des revendications 1 à 6, dans lequel la section transparente (2, 52) comporte une section adhésive transparente (9, 2b, 52b) sur une surface de la section transparente (2, 52) qui fait face à la surface corporelle (BS) lorsque l'élément de couverture en forme de feuille (1, 51) est fixé à la surface corporelle (BS).

8. Élément de couverture en forme de feuille (1, 51) selon l'une quelconque des revendications 1 à 7, dans lequel la feuille thermosensible (70) comporte, sur une surface de la feuille thermosensible (70) tournée face à la feuille de résine, une section adhésive (53f) qui adhère à la feuille de résine.

9. Élément de couverture en forme de feuille (1, 51) selon la revendication 8, dans lequel la feuille thermosensible (70) comporte une poignée (72) pouvant être saisie lors du décollement de la section adhésive (53f) de la feuille de résine.

10. Élément de couverture en forme de feuille (1, 51) selon la revendication 8 ou 9, dans lequel
la section adhésive (53f) de la feuille thermosensible (70) est désignée comme une première section adhésive, et une seconde section adhésive (53d) capable d'adhérer à la surface corporelle (BS) est disposée sur la feuille de résine, et
une force adhésive de la première section adhésive (53f) par rapport à la feuille de résine est inférieure à une force adhésive de la seconde section adhésive (53d) par rapport à la surface corporelle (BS).
